# EUROPEAN PATENT APPLICATION

(11) **EP 2 286 816 A1**
(43) Date of publication of application: **23.02.2011**
(21) Application number: 10182413.4
(22) Date of filing: 13.05.2004
(51) Int. Cl.: A61K 31/455, A61K 31/00, A61K 31/403, A61K 31/437, A61K 31/538, A61P 3/06

(54) **Method of treating atherosclerosis, dyslipidemias and related conditions**

(30) Priority: 15.05.2003 US 470665 P
(62) Divisional of application: 09163448.5
(71) Applicant: MERCK SHARP & DOHME (I.A.) CORP., Rahway, New Jersey 07065 (US); Merck Frosst Canada & Co., Kirkland, Quebec H9H 3L1 (CA)
(72) Inventor: Cheng, Kang, Rahway, NJ 07065-0907 (US); O'Neil, Gary, Kirkland Québec H9H 3L1 (CA); Waters, Gerard, Rahway, NJ 07065-0907 (US); Metters, Kathleen, Kirkland Québec H9H 3L1 (CA)
(74) Representative: Hussain, Deeba

(57) **Abstract**

A combination of a DP antagonist and niacin for use in therapy.

## Description

### CROSS-REFERENCE TO RELATED APPLICATIONS

The present invention is related to U.S. provisional application Serial no. 60/470,665, filed May 15, 2003, the contents of which are hereby incorporated by reference.

### BACKGROUND OF THE INVENTION

Niacin or nicotinic acid (pyridine-3-carboxylic acid) is a drug commonly known for its effect in elevating serum levels of high density lipoproteins (HDL). However, nicotinic acid is frequently associated with cutaneous vasodilation, sometimes called flushing. This side effect is caused by the nicotinic acid-induced release of prostaglandin D2 in the skin and is so severe that many patients discontinue nicotinic acid treatment. The present invention relates to the treatment of atherosclerosis, dyslipidemias, diabetes and related conditions by administering nicotinic acid or another nicotinic acid receptor agonist in combination with a compound that reduces or eliminates the cutaneous vasodilation that otherwise occurs, such that treatment can progress without substantial flushing. This is achieved in humans by administering nicotinic acid or a nicotinic acid receptor agonist and a compound that antagonizes the DP receptor.

Different subtypes of receptors interact with prostaglandin D2. One prostaglandin D2 receptor is referred to as "DP" and another prostaglandin D2 receptor is known as "CRTH2". The present invention utilizes antagonism of the DP receptor to prevent, minimize or reduce flushing that otherwise may occur.

Consequently one object of the present invention is to eliminate or reduce substantial flushing (frequency and/or severity) as a side effect during the treatment of humans for atherosclerosis, dyslipidemia, diabetes and related conditions using nicotinic acid or another nicotinic acid receptor agonist.

Another object of the present invention is to provide combination therapy for atherosclerosis that minimizes side effects generally.

Yet another object is to provide a fixed combination pharmaceutical composition for oral use.

These and other objects will be apparent from the description provided herein.

### SUMMARY OF THE INVENTION

A method of treating atherosclerosis in a human patient in need of such treatment is provided that is comprised of administering to the patient nicotinic acid or a salt or solvate thereof, or another nicotinic acid receptor agonist and a DP receptor antagonist in amounts that are effective for treating atherosclerosis in the absence of substantial flushing.

### BRIEF DESCRIPTION OF THE DRAWINGS

The invention is illustrated in connection with the figures appended hereto in which:
Figure 1 is a graph that shows that Compound D inhibits prostaglandin D2-induced vasodilation in mice;
Figure 2 is a graph that shows that Compound D inhibits nicotinic acid induced vasodilation in mice.
Figure 3 is a graph that shows that other selected compounds inhibit nicotinic acid-induced vasodilation in mice.

### DETAILED DESCRIPTION OF THE INVENTION

Niacin or nicotinic acid (pyridine-3-carboxylic acid) is a drug commonly known for its effect in the elevation of high density lipoproteins (HDL) levels, as well as other beneficial alterations of the lipid profile (lowering very low density (VLDL), low density lipoprotein (LDL), triglycerides, free fatty acids (FFA) and lipoprotein(a) [Lp(a)]). Nicotinic acid raises HDL levels when administered to humans in therapeutically effective doses, such as about 50 mg to as high as about 8 grams per day. However, nicotinic acid is frequently associated with cutaneous vasodilation, also called flushing. Flushing typically entails a reddening of the skin, accompanied by warmth, itchiness or irritation. It can be extremely unpleasant, and can be so severe that many patients discontinue nicotinic acid treatment. The present invention relates to the treatment, prevention or reversal of atherosclerosis and the other diseases and conditions described herein, with nicotinic acid or a salt or solvate thereof, or another nicotinic acid receptor agonist without substantial flushing. This is achieved in humans by administering nicotinic acid or a salt or solvate thereof, or another nicotinic acid receptor agonist and a compound that antagonizes the DP receptor, thus preventing, reducing or minimizing the flushing effect in it frequency and/or severity.

There are at least two receptors that interact with prostaglandin D2, referred to as "DP" and "CRTH2". The present invention is primarily concerned with nicotinic acid or nicotinic acid receptor agonists used in combination with antagonists of the DP receptor.

One aspect of the invention that is of interest is a method of treating atherosclerosis in a human patient in need of such treatment comprising administering to the patient nicotinic acid or a salt or solvate thereof, or another nicotinic acid receptor agonist and a DP receptor antagonist in amounts that are effective for treating atherosclerosis in the absence of substantial flushing.

Another aspect of the invention that is of interest relates to a method of raising serum HDL levels in a human patient in need of such treatment, comprising administering to the patient nicotinic acid or a salt or solvate thereof, or another nicotinic acid receptor agonist and a DP receptor antagonist, said combination being effective for raising serum HDL levels in the patient in the absence of substantial flushing.

Another aspect of the invention that is of interest relates to a method of treating dyslipidemia in a human patient in need of such treatment comprising administering to the patient nicotinic acid or a salt or solvate thereof, or another nicotinic acid receptor agonist and a DP receptor antagonist in amounts that are effective for treating dyslipidemia in the absence of substantial flushing.

Another aspect of the invention that is of interest relates to a method of reducing serum VLDL or LDL levels in a human patient in need of such treatment, comprising administering to the patient nicotinic acid or a salt or solvate thereof, or another nicotinic acid receptor agonist and a DP receptor antagonist, in amounts that are effective for reducing serum VLDL or LDL levels in the patient in the absence of substantial flushing.

Another aspect of the invention that is of interest relates to a method of reducing serum triglyceride levels in a human patient in need of such treatment, comprising administering to the patient nicotinic acid or a salt or solvate thereof, or another nicotinic acid receptor agonist and a DP receptor antagonist, in amounts that are effective for reducing serum triglyceride levels in the patient in the absence of substantial flushing.

Another aspect of the invention that is of interest relates to a method of reducing serum Lp(a) levels in a human patient in need of such treatment, comprising administering to the patient nicotinic acid or a salt or solvate thereof, or another nicotinic acid receptor agonist and a DP receptor antagonist, in amounts that are effective for reducing serum Lp(a) levels in the patient in the absence of substantial flushing. As used herein Lp(a) refers to lipoprotein (a).

An aspect of the invention that is of particular interest relates to each of the methods described above wherein nicotinic acid or a salt or solvate thereof is utilized. More particularly of interest is the use of nicotinic acid. In yet a further aspect that is of interest, the DP receptor antagonist selectively modulates the DP receptor in amounts that are effective for reducing or preventing the flushing effect in the patient.

Another aspect of the invention that is of particular interest relates to each of the methods described above wherein nicotinic acid is utilized and the DP receptor antagonist selectively modulates the DP receptor and does not substantially modulate the CRTH2 receptor.

Another aspect of the invention that is of particular interest relates to a method of treating atherosclerosis, dyslipidemias, diabetes or a related condition in a human patient in need of such treatment, comprising administering to the patient nicotinic acid or a salt or solvate thereof, or another nicotinic acid receptor agonist and a DP receptor antagonist, said combination being administered in an amount that is effective to treat atherosclerosis, dyslipidemia, diabetes or a related condition in the absence of substantial flushing.

One aspect of the invention is the use of a DP receptor antagonist compound in combination with nicotinic acid or a salt or solvate thereof, or another nicotinic acid receptor agonist for treating atherosclerosis in a human in the absence of substantial flushing.

Another aspect of the invention that is of particular interest relates to the methods described above wherein the DP receptor antagonist is selected from the group consisting of compounds A through AJ and the pharmaceutically acceptable salts and solvates thereof.

Examples of compounds that are particularly useful for selectively antagonizing DP receptors and suppressing the flushing effect include the following:

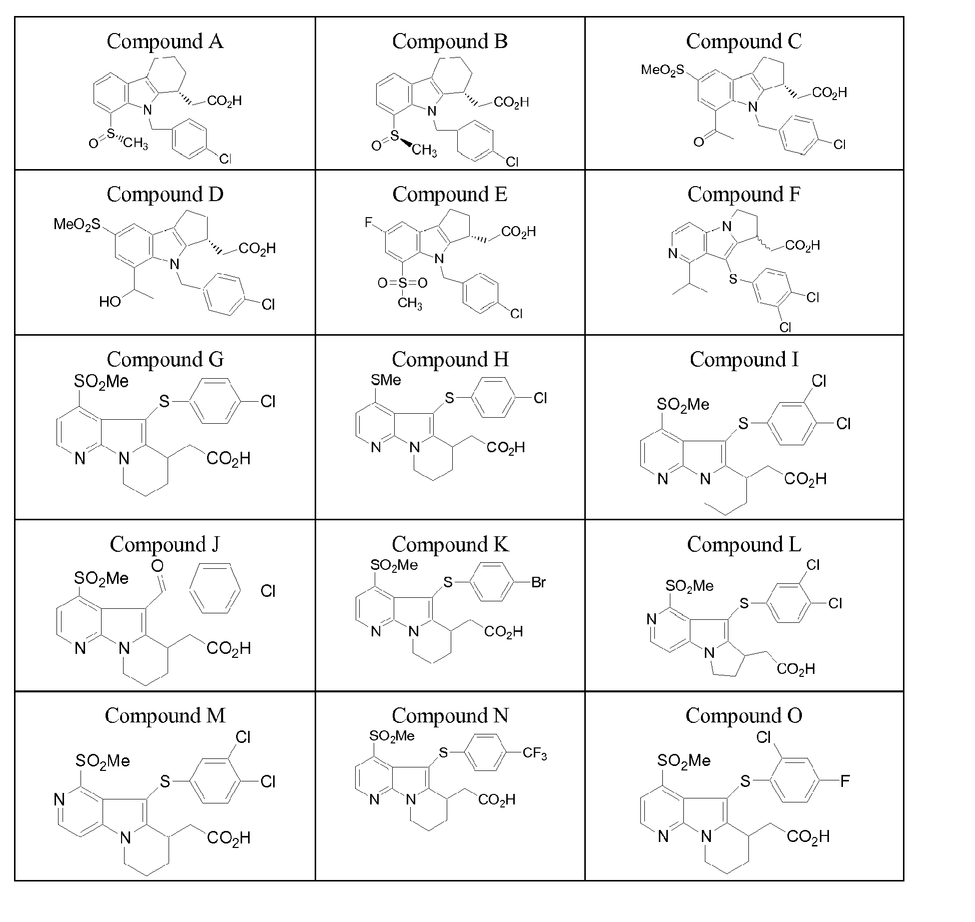

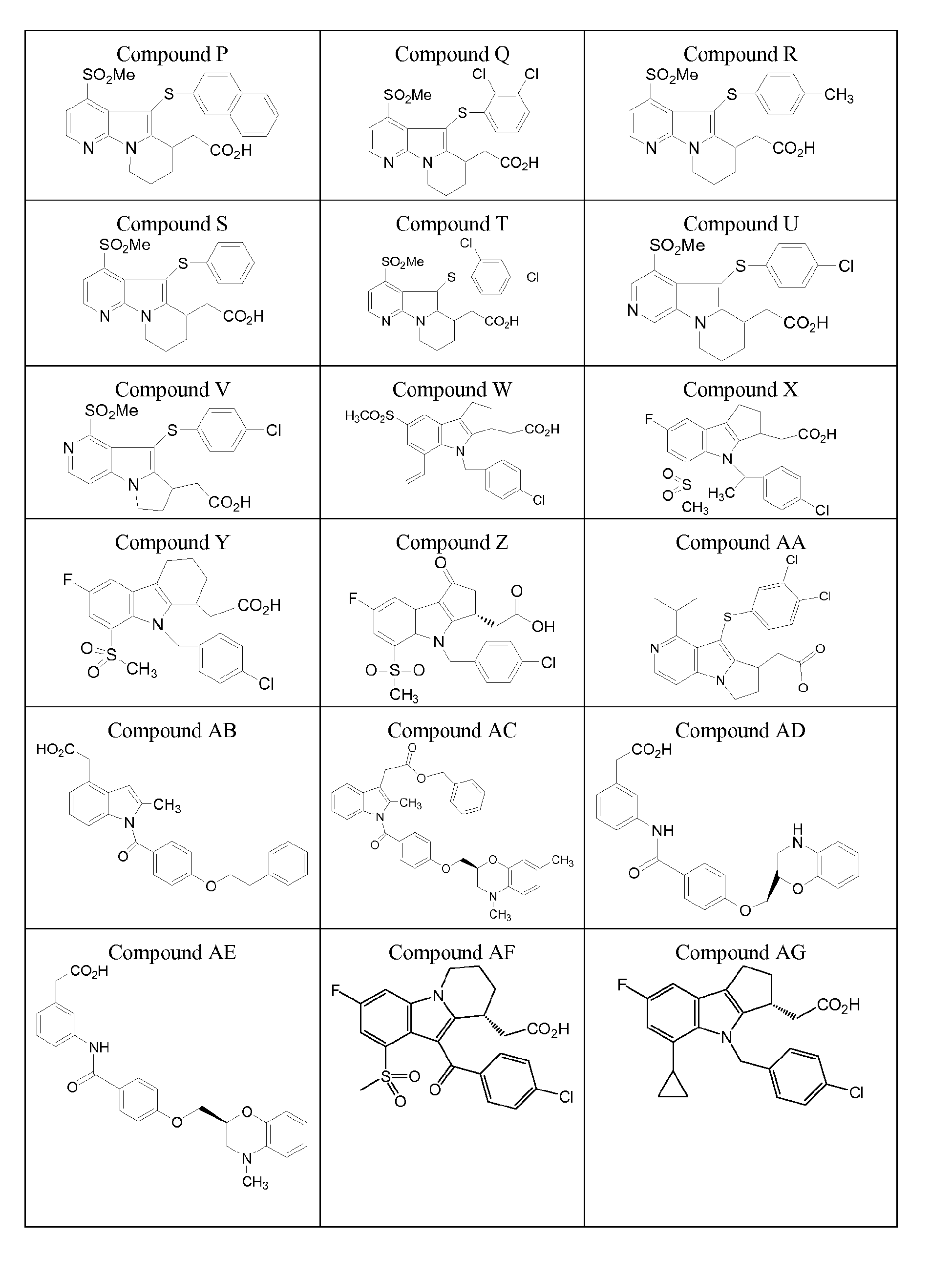

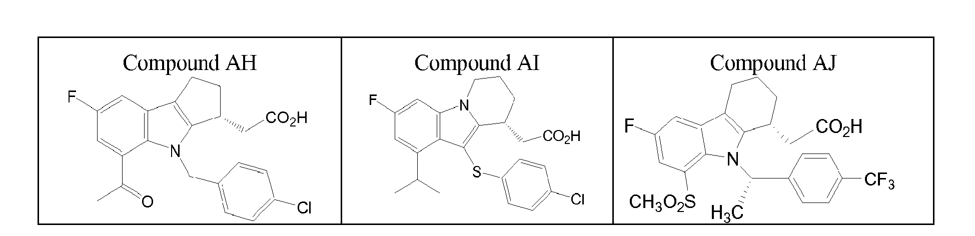

as well as the pharmaceutically acceptable salts and solvates thereof.

Atherosclerosis as used herein refers to a form of vascular disease characterized by the deposition of atheromatous plaques containing cholesterol and lipids on the innermost layer of the walls of large and medium-sized arteries. Atherosclerosis encompasses vascular diseases and conditions that are recognized and understood by physicians practicing in the relevant fields of medicine. Atherosclerotic cardiovascular disease, including restenosis following revascularization procedures, coronary heart disease (also known as coronary artery disease or ischemic heart disease), cerebrovascular disease including multi-infarct dementia, and peripheral vessel disease including erectile dysfunction, are all clinical manifestations of atherosclerosis and are therefore encompassed by the terms "atherosclerosis" and "atherosclerotic disease."

"Dyslipidemia" is used in the conventional sense to refer to abnormal levels of plasma lipids, such as HDL (low), LDL (high), VLDL (high), triglycerides (high), lipoprotein (a) (high), FFA (high) and other serum lipids, or combinations thereof. It may be an uncomplicated condition or part of a particular related disease or condition such as diabetes (diabetic dyslipidemia), metabolic syndrome and the like. Thus, uncomplicated dyslipidemias as well as those that are associated with underlying conditions are included in the present invention.

The term "patient" includes mammals, especially humans, who use the instant active agents for the prevention or treatment of a medical condition. Administering the drugs to the patient includes both self-administration and administration to the patient by another person. The patient may be in need of treatment for an existing disease or medical condition, or may desire prophylactic treatment to prevent or reduce the risk of onset of atherosclerosis.

The term "therapeutically effective amount" is intended to mean that amount of drug that will elicit the desired biological or medical response. As an example, nicotinic acid is often administered at doses from about 50 mg to about 8 grams each day.

The terms "prophylactically effective amount" and "amount that is effective to prevent" refer to that amount of drug that will prevent or reduce the risk of occurrence of the biological or medical event that is sought to be prevented. In many instances, the prophylactically effective amount is the same as the therapeutically effective amount.

The invention described herein includes the administration of the compounds and compositions described herein to prevent or reduce the risk of occurrence, or recurrence where the potential exists, of a coronary heart disease event, a cerebrovascular event, and/or intermittent claudication. Coronary heart disease events are intended to include CHD death, myocardial infarction (i.e., a heart attack), and coronary revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. The term "atherosclerotic disease event" as used herein is intended to encompass coronary heart disease events, cerebrovascular events, and intermittent claudication experienced one or more non-fatal atherosclerotic disease events are those for whom the potential for recurrence of such an event exists.. It is intended that persons who have previously

Accordingly, the instant invention also provides a method for preventing or reducing the risk of a first or subsequent occurrence of an atherosclerotic disease event comprising the administration of a prophylactically effective amount of the compounds described herein to a patient at risk for such an event while preventing or minimizing substantial flushing. The patient may already have atherosclerotic disease at the time of administration, or may be at risk for developing it.

The method further relates to preventing or slowing new atherosclerotic lesion or plaque formation, and preventing or slowing the progression of existing lesions or plaques, as well as to causing the regression of existing lesions or plaques, while preventing or minimizing substantial flushing.

Accordingly, one aspect of this invention involves a method for halting or slowing the progression of atherosclerosis, including halting or slowing atherosclerotic plaque progression, comprising administering a therapeutically effective amount of any of the DP antagonists described herein in combination with nicotinic acid or another nicotinic acid receptor agonist to a patient in need of such treatment. This method also includes halting or slowing progression of atherosclerotic plaques existing at the time the instant treatment is begun (i.e., "existing atherosclerotic plaques"), as well as halting or slowing formation of new atherosclerotic plaques in patients with atherosclerosis.

Another aspect of this invention involves a method for preventing or reducing the risk of atherosclerotic plaque rupture comprising administering a prophylactically effective amount of any of the compounds described herein along with nicotinic acid or another nicotinic acid receptor agonist to a patient in need of such treatment. Rupture as used herein refers to the breaking loose of plaque, which can become lodged in blood vessels. A further aspect of this invention involves a method for preventing or reducing the risk of developing atherosclerosis, comprising administering a prophylactically effective amount of the compounds described herein to a patient in need of such treatment.

Another aspect of the invention relates to a method of treating or preventing atherosclerosis, dyslipidemias or a related condition comprising pretreating a human patient in need of such therapy with a flush-inhibiting or reducing effective amount of a DP receptor antagonist, thereafter treating said patient with nicotinic acid, a salt or solvate thereof, or another nicotinic acid receptor agonist in an amount that is effective to treat or prevent said atherosclerosis, dyslipidemia or related condition in the absence of substantial flushing.

Yet another aspect of the invention relates to the method described above, further comprising pre-treating or treating the patient with an HMG Co-A reductase inhibitor.

Another aspect of the invention relates to a method of treating or preventing the conditions noted above wherein the HMG Co-A reductase inhibitor is simvastatin.

One aspect of the methods described herein relates to the use of nicotinic acid or another nicotinic acid receptor agonist compound in an amount that is effective for achieving the results described herein, and a DP receptor antagonist that selectively modulates the DP receptor without substantially modulating the CRTH2 receptor. Thus, the DP receptor antagonist has an affinity at the DP receptor (i.e., Kᵢ) that is at least about 10 times higher (a numerically lower Kᵢ value) than the affinity at the CRTH2 receptor. Any compound that selectively interacts with DP according to these guidelines is deemed "DP selective".

The phrase "in the absence of substantial flushing" refers to the side effect that is often seen when nicotinic acid is administered in therapeutic amounts. The flushing effect of nicotinic acid usually becomes less frequent and less severe as the patient develops tolerance to the drug at therapeutic doses, but the flushing effect still occurs to some extent. Thus, "in the absence of substantial flushing" refers to the reduced severity of flushing when it occurs, or fewer flushing events than would otherwise occur. Preferably, the incidence of flushing is reduced by at least about a third, more preferably the incidence is reduced by half, and most preferably, the flushing incidence is reduced by about two thirds or more. Likewise, the severity is preferably reduced by at least about a third, more preferably by at least half, and most preferably by at least about two thirds. Clearly a one hundred percent reduction in flushing incidence and severity is most preferable, but is not required.

The specific dosage regimen and levels for any particular patient will depend upon a variety of factors including the age, body weight, general health, sex, diet, time of administration, route of administration, rate of excretion, drug combination and the severity of the patient's condition. Consideration of these factors is well within the purview of the ordinarily skilled clinician for the purpose of determining the therapeutically effective or prophylactically effective dosage amount needed to prevent, counter, or arrest the progress of the condition. It is expected that the compounds described herein will be administered on a daily basis for a length of time appropriate to treat or prevent the medical condition relevant to the patient, including a course of therapy lasting months, years or the life of the patient.

One or more additional active agents may be administered with the compounds described herein. The additional active agent or agents can be lipid modifying compounds or agents having other pharmaceutical activities, or agents that have both lipid-modifying effects and other pharmaceutical activities. Examples of additional active agents which may be employed include but are not limited to HMG-CoA reductase inhibitors, which include statins in their lactonized or dihydroxy open acid forms and pharmaceutically acceptable salts and esters thereof, including but not limited to lovastatin (see US Patent No. 4,342,767), simvastatin (see US Patent No. 4,444,784), dihydroxy open-acid simvastatin, particularly the ammonium or calcium salts thereof, pravastatin, particularly the sodium salt thereof (see US Patent No. 4,346,227), fluvastatin particularly the sodium salt thereof (see US Patent No. 5,354,772), atorvastatin, particularly the calcium salt thereof (see US Patent No. 5,273,995), pitavastatin also referred to as NK-104 (see PCT international publication number WO 97/23200) and rosuvastatin, also known as ZD-4522, (CRESTOR^{®}; see US Patent No. 5,260,440); HMG-CoA synthase inhibitors; squalene epoxidase inhibitors; squalene synthetase inhibitors (also known as squalene synthase inhibitors), acyl-coenzyme A: cholesterol acyltransferase (ACAT) inhibitors including selective inhibitors of ACAT-1 or ACAT-2 as well as dual inhibitors of ACAT-1 and -2; microsomal triglyceride transfer protein (MTP) inhibitors; endothelial lipase inhibitors; bile acid sequestrants; LDL receptor inducers; platelet aggregation inhibitors, for example glycoprotein IIb/IIIa fibrinogen receptor antagonists and aspirin; human peroxisome proliferator activated receptor gamma (PPARγ) agonists including the compounds commonly referred to as glitazones for example pioglitazone and rosiglitazone and, including those compounds included within the structural class known as thiazolidine diones as well as those PPARγ agonists outside the thiazolidine dione structural class; PPARα agonists such as clofibrate, fenofibrate including micronized fenofibrate, and gemfibrozil; PPAR dual α/γ agonists; vitamin B₆ (also known as pyridoxine) and the pharmaceutically acceptable salts thereof such as the HCl salt; vitamin B₁₂ (also known as cyanocobalamin); folic acid or a pharmaceutically acceptable salt or ester thereof such as the sodium salt and the methylglucamine salt; anti-oxidant vitamins such as vitamin C and E and beta carotene; beta-blockers; angiotensin II antagonists such as losartan; angiotensin converting enzyme inhibitors such as enalapril and captopril; renin inhibitors, calcium channel blockers such as nifedipine and diltiazem; endothelin antagonists; agents that enhance ABCA1 gene expression; cholesteryl ester transfer protein (CETP) inhibiting compounds, 5-lipoxygenase activating protein (FLAP) inhibiting compounds, 5-lipoxygenase (5-LO) inhibiting compounds, farnesoid X receptor (FXR) ligands including both antagonists and agonists; Liver X Receptor (LXR)-alpha ligands, LXR-beta ligands, bisphosphonate compounds such as alendronate sodium; cyclooxygenase-2 inhibitors such as rofecoxib and celecoxib; and compounds that attenuate vascular inflammation.

Cholesterol absorption inhibitors can also be used in the present invention. Such compounds block the movement of cholesterol from the intestinal lumen into enterocytes of the small intestinal wall, thus reducing serum cholesterol levels. Examples of cholesterol absorption inhibitors are described in U.S. Patent Nos. 5,846,966, 5,631,365, 5,767,115, 6,133,001, 5,886,171, 5,856,473, 5,756,470, 5,739,321, 5,919,672, and in PCT application Nos. WO 00/63703, WO 00/60107, WO 00/38725, WO 00/34240, WO 00/20623, WO 97/45406, WO 97/16424, WO 97/16455, and WO 95/08532. The most notable cholesterol absorption inhibitor is ezetimibe, also known as 1-(4-fluorophenyl)-3(R)-[3(S)-(4-fluorophenyl)-3-hydroxypropyl)]-4(S)-(4-hydroxyphenyl)-2-azetidinone, described in U.S. Patent Nos. 5,767,115 and 5,846,966.

Therapeutically effective amounts of cholesterol absorption inhibitors include dosages of from about 0.01 mg/kg to about 30 mg/kg of body weight per day, preferably about 0.1 mg/kg to about 15 mg/kg.

For diabetic patients, the compounds used in the present invention can be administered with conventional diabetic medications. For example, a diabetic patient receiving treatment as described herein may also be taking insulin or an oral antidiabetic medication. One example of an oral antidiabetic medication useful herein is metformin.

### Dosage Information

Nicotinic acid as used herein refers to pyridine-3-carboxylic acid. However, salts and solvates of nicotinic acid are also included for use in the present invention, and numerous pharmaceutically acceptable salts and solvates of nicotinic acid are useful in the present invention. Alkali metal salts, in particular, sodium and potassium, form salts that are useful as described herein. Likewise alkaline earth metals, in particular, calcium and magnesium, form salts that are useful as described herein. Various salts of amines, such as ammonium and substituted ammonium compounds also form salts that are useful as described herein. Similarly, solvated forms of nicotinic acid are useful within the present invention. Examples include the hemihydrate, mono-, di-, tri- and sesquihydrate. Of particular interest for use in the present invention is the free acid, pyridine-3-carboxylic acid.

DP antagonists, as described herein, are useful for reducing or preventing the flushing effect in mammalian patients, particularly humans, at dosages ranging from as low as about 0.01 mg/kg/day to as high as about 100 mg/kg/day, administered in single or divided daily doses. Preferably the dosages are from about 0.1 mg/day to as high as about 1.0 g/day, in single or divided daily doses.

The dose of nicotinic acid that is useful as described herein ranges from as low as about 50 mg/day to as high as about 8 g/day, in single or divided daily doses. Lower dosages can be used initially, and dosages increased to further minimize the flushing effect.

The dosages of nicotinic acid receptor agonists other than nicotinic acid vary within wide limits. Generally, nicotinic acid receptor agonists that are useful for treating atherosclerosis will be administered in amounts ranging from as low as about 0.01 mg/kg/day to as high as about 100 mg/kg/day, in single or divided doses. A representative dosage is about 0.1 mg/day to about 2 g/day.

The compounds used in the present invention can be administered via any conventional route of administration. The preferred route of administration is oral.

The nicotinic acid, salt or solvate thereof, or other nicotinic acid receptor agonist and the DP antagonist can be administered together or sequentially in single or multiple daily doses, e.g., bid, tid or qid, without departing from the invention. If particularly long sustained release is desired, such as a sustained release product showing a release profile that extends beyond 24 hours, dosages may be administered every other day. However, single daily doses are preferred. Likewise, morning or evening dosages can be utilized.

### Pharmaceutical Compositions

The pharmaceutical compositions described herein are generally comprised of nicotinic acid or another nicotinic acid receptor agonist, a DP receptor antagonist and a pharmaceutically acceptable carrier.

Examples of suitable oral compositions include tablets, capsules, troches, lozenges, suspensions, dispersible powders or granules, emulsions, syrups and elixirs. Examples of carrier ingredients include diluents, binders, disintegrants, lubricants, sweeteners, flavors, colorants, preservatives, and the like. Examples of diluents include, for example, calcium carbonate, sodium carbonate, lactose, calcium phosphate and sodium phosphate. Examples of granulating and disintegrants include corn starch and alginic acid. Examples of binding agents include starch, gelatin and acacia. Examples of lubricants include magnesium stearate, calcium stearate, stearic acid and talc. The tablets may be uncoated or coated by known techniques. Such coatings may delay disintegration and thus, absorption in the gastrointestinal tract and thereby provide a sustained action over a longer period.

In one embodiment of the invention, nicotinic acid, a salt or solvate thereof, or another nicotinic acid receptor agonist is combined with the DP receptor antagonist and the carrier to form a fixed combination product. This fixed combination product may be a tablet or capsule for oral use.

More particularly, in another embodiment of the invention, nicotinic acid, or a salt or solvate thereof, or another nicotinic acid receptor agonist (about 1 to about 1000 mg) and the DP antagonist (about 1 to about 500 mg) are combined with the pharmaceutically acceptable carrier, providing a tablet or capsule for oral use.

Sustained release over a longer period of time may be particularly important in the formulation of nicotinic acid pharmaceutical compositions. Sustained release tablets are particularly preferred. For example, a time delay material such as glyceryl monostearate or glyceryl distearate may be employed. The dosage form may also be coated by the techniques described in the U.S. Patent Nos. 4,256,108; 4,166,452 and 4,265,874 to form osmotic therapeutic tablets for controlled release.

Other controlled release technologies are also available and are included herein. Typical ingredients that are useful to slow the release of nicotinic acid in sustained release tablets include various cellulosic compounds, such as methylcellulose, ethylcellulose, propylcellulose, hydroxypropylcellulose, hydroxyethylcellulose, hydroxypropylmethylcellulose, microcrystalline cellulose, starch and the like. Various natural and synthetic materials are also of use in sustained release formulations. Examples include alginic acid and various alginates, polyvinyl pyrrolidone, tragacanth, locust bean gum, guar gum, gelatin, various long chain alcohols, such as cetyl alcohol and beeswax.

A sustained release tablet that is of particular interest utilizes nicotinic acid in combination with one or more of the cellulosic compounds noted above, compressed into a sustained release tablet to form a polymer matrix. The DP antagonist compound can be incorporated into the blend before compression, or can be coated onto the outer surface of the matrix.

In an embodiment that is of more interest, the nicotinic acid and matrix-forming material are combined and compressed to form a sustained release core, and the DP antagonist compound is blended with one or more coating agents and coated onto the outer surface of the core.

Optionally and of even more interest is a tablet as described above, further coated with an HMG Co-A reductase inhibitor, for example, simvastatin. This particular embodiment thus contains three active ingredients, the HMG Co-A reductase inhibitor and the DP antagonist, which may be releasable substantially upon ingestion, and the nicotinic acid which may be releasable over a longer period of time as described above.

Typical release time frames for sustained release tablets in accordance with the present invention range from about 1 to as long as about 48 hours, preferably about 4 to about 24 hours, and more preferably about 8 to about 16 hours.

Hard gelatin capsules constitute another solid dosage form for oral use. Such capsules similarly include the active ingredients mixed with carrier materials as described above. Soft gelatin capsules include the active ingredients mixed with water-miscible solvents such as propylene glycol, PEG and ethanol, or an oil such as peanut oil, liquid paraffin or olive oil.

Aqueous suspensions are also contemplated as containing the active material in admixture with excipients suitable for the manufacture of aqueous suspensions. Such excipients include suspending agents, for example sodium carboxymethylcellulose, methylcellulose, hydroxypropylmethylcellulose, sodium alginate, polyvinylpyrrolidone, tragacanth and acacia; dispersing or wetting agents,e.g., lecithin; preservatives, e.g., ethyl, or n-propyl para-hydroxybenzoate, colorants, flavors, sweeteners and the like.

Dispersible powders and granules suitable for preparation of an aqueous suspension by the addition of water provide the active ingredients in admixture with a dispersing or wetting agent, suspending agent and one or more preservatives. Suitable dispersing or wetting agents and suspending agents are exemplified by those already mentioned above.

Syrups and elixirs may also be formulated.

The pharmaceutical composition that is of particular interest is a sustained release tablet that is comprised of nicotinic acid or a salt or solvate thereof, and a DP receptor antagonist in combination with a pharmaceutically acceptable carrier.

Another pharmaceutical composition that is of particular interest is a sustained release tablet that is comprised of nicotinic acid or a salt or solvate thereof,, a DP receptor antagonist and an HMG Co-A reductase inhibitor in combination with a pharmaceutically acceptable carrier.

Yet another pharmaceutical composition that is of more particular interest is a sustained release tablet that is comprised of nicotinic acid, a DP receptor antagonist and simvastatin in combination with a pharmaceutically acceptable carrier.

Yet another pharmaceutical composition that is of particular interest is a sustained release tablet that is comprised of nicotinic acid, and a DP receptor antagonist that is selected from the group consisting of compounds A through AJ in combination with a pharmaceutically acceptable carrier.

Yet another pharmaceutical composition that is of more interest is comprised of nicotinic acid and a DP antagonist compound selected from the group consisting of compounds A, B, D, E, X, AA, AF, AG, AH, AI and AJ, in combination with a pharmaceutically acceptable carrier.

Yet another pharmaceutical composition that is of particular interest is comprised of nicotinic acid and DP antagonist compound A in combination with a pharmaceutically acceptable carrier.

Yet another pharmaceutical composition that is of more interest is comprised of nicotinic acid and DP antagonist compound B in combination with a pharmaceutically acceptable carrier.

Yet another pharmaceutical composition that is of more interest is comprised of nicotinic acid and DP antagonist compound D in combination with a pharmaceutically acceptable carrier.

Yet another pharmaceutical composition that is of more interest is comprised of nicotinic acid and DP antagonist compound E in combination with a pharmaceutically acceptable carrier.

Yet another pharmaceutical composition that is of more interest is comprised of nicotinic acid and DP antagonist compound X in combination with a pharmaceutically acceptable carrier.

Yet another pharmaceutical composition that is of more interest is comprised of nicotinic acid and DP antagonist compound AA in combination with a pharmaceutically acceptable carrier.

Yet another pharmaceutical composition that is of more interest is comprised of nicotinic acid and DP antagonist compound AF in combination with a pharmaceutically acceptable carrier.

Yet another pharmaceutical composition that is of more interest is comprised of nicotinic acid and DP antagonist compound AG in combination with a pharmaceutically acceptable carrier.

Yet another pharmaceutical composition that is of more interest is comprised of nicotinic acid and DP antagonist compound AH in combination with a pharmaceutically acceptable carrier.

Yet another pharmaceutical composition that is of more interest is comprised of nicotinic acid and DP antagonist compound AI in combination with a pharmaceutically acceptable carrier.

Yet another pharmaceutical composition that is of more interest is comprised of nicotinic acid and DP antagonist compound AJ in combination with a pharmaceutically acceptable carrier.

Yet another pharmaceutical composition that is of even more interest is comprised of nicotinic acid, one of the DP antagonist compounds noted above and simvastatin in combination with a pharmaceutically acceptable carrier.

Yet another pharmaceutical composition that is of more interest is a sustained release tablet that is comprised of nicotinic acid, a DP receptor antagonist that is selected from the group consisting of compounds A through AJ and simvastatin in combination with a pharmaceutically acceptable carrier.

Yet another pharmaceutical composition that is of more particular interest relates to a sustained release tablet that is comprised of nicotinic acid, a DP receptor antagonist selected from the group consisting of compounds A, B, D, E, X, AA, AF, AG, AH, AI and AJ, and simvastatin in combination with a pharmaceutically acceptable carrier.

The term "composition", in addition to encompassing the pharmaceutical compositions described above, also encompasses any product which results, directly or indirectly, from the combination, complexation or aggregation of any two or more of the ingredients, active or excipient, or from dissociation of one or more of the ingredients, or from other types of reactions or interactions of one or more of the ingredients. Accordingly, the pharmaceutical composition of the present invention encompasses any composition made by admixing or otherwise combining the compounds, any additional active ingredient(s), and the pharmaceutically acceptable excipients.

Another aspect of the invention relates to the use of nicotinic acid or a salt or solvate thereof, or another nicotinic acid receptor agonist and a DP antagonist in the manufacture of a medicament. This medicament has the uses described herein.

More particularly, another aspect of the invention relates to the use of nicotinic acid or a salt or solvate thereof, or another nicotinic acid receptor agonist, a DP antagonist and an HMG Co-A reductase inhibitor, such as simvastatin, in the manufacture of a medicament. This medicament has the uses described herein.

In addition to nicotinic acid, which is the benchmark nicotinic acid receptor agonist, numerous nicotinic acid receptor agonists have been described. The following publications disclose compounds that are nicotinic acid receptor agonists:
Lorenzen, A. et al. Molecular Pharmacology 59: 349-357 (2001),
Lorenzen, A. et al. Biochemical Pharmacology 64: 645-648 (2002),
Soga, T. et al. Biochemical and Biophysical Research Comm. 303: 364-369 (2003),
Tunaru, S. et al. Nature Medicine 9: 352-355 (2003),
Wise, A. et al. Journal of Biological Chemistry 278: 9869-9874 (2003), and
Van Herk, T. et al Journal of Medicinal Chemistry 46: 3945-3951 (2003).

It is noted that partial agonists for the nicotinic acid receptor, such as those disclosed in van Herk, et al. are included in the present compositions and methods of treatment.

Moreover, the nicotinic acid receptor has been identified and characterized in WO02/084298A2 published on October 24, 2002 and in Soga, T. et al., Tunaru, S. et al. and Wise, A. et al. (citations above).

Numerous DP receptor antagonist compounds have been published and are useful and included in the methods of the present invention. For example, DP receptor antagonists can be obtained in accordance with WO01/79169 published on October 25, 2001, EP 1305286 published on May 2, 2003, WO02/094830 published on November 28, 2002 and WO03/062200 published on July 31, 2003. Compound AB can be synthesized in accordance with the description set forth in WO01/66520A1 published on September 13, 2001; Compound AC can be synthesized in accordance with the description set forth in WO03/022814A1 published on March 20, 2003, and Compounds AD and AE can be synthesized in accordance with the description set forth in WO03/078409 published on September 25, 2003. Other representative DP antagonist compounds used in the present invention can be synthesized in accordance with the examples provided below.

### EXAMPLE 1

### [5-[(4-Chlorophenyl)thio]-4-(methylsulfonyl)-6,7,8,9-tetrahydropyrido[3,2-b]indolizin-6-yl]acetic acid (Compound G)

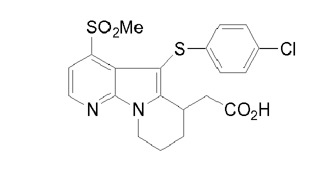

### Step 1 4-Chloronicotinaldehyde

The title compound was prepared as described by F. Marsais et al., J. Heterocyclic Chem., 25,81 (1988).

### Step 2 4-(Methylthio)nicotinaldehyde

To a solution of NaSMe (9.5 g, 135 mmol) in MeOH (250 mL) was added the 4-chloronicotinaldehyde (13.5 g, 94.4 mmol) of Step 1 in MeOH (250 mL). The reaction mixture was maintained at 60°C for 15 min. The reaction mixture was poured over NH₄Cl and EtOAc. The organic phase was separated, washed with H₂O and dried over Na₂SO₄. The compound was then purified over silica gel with 50% EtOAc in Hexanes to provide the title compound.

### Step 3 Methyl (2Z)-2-azido-3-[4-(methylthio)pyridin-3-yl]prop-2-enoate

A solution of 4-(methylthio)nicotinealdehyde (4.8 g, 31 mmol) and methyl azidoacetate (9.0 g, 78 mmol) in MeOH (50 mL) was added to a solution of 25% NaOMe in MeOH (16.9 mL, 78 mmol) at -12°C. The internal temperature was monitored and maintained at -10°C to -12°C during the 30 min. addition. The resulting mixture was then stirred in an ice bath for several hours, followed by overnight in an ice bath in the cold room. The suspension was then poured onto a mixture of ice and NH₄Cl, and the slurry was filtered after 10 min. of stirring. The product was washed with cold H₂O and was then dried under vacuum to give the title compound as a beige solid (7.4 g), which contained some salts.The compound is then purified over silica gel with EtOAc.

### Step 4 Methyl 4-(methylthio)-1H-pyrrolo[2,3-b]pyridine-2-carboxylate

A suspension of the compound of Step 3 (0.40 g, 1.6 mmol) in xylenes (16 mL) was heated slowly to 140°C. After a period of 15 min. at 140°C, the yellow solution was cooled to room temperature. Precaution must be taken due to the possibility of an exotherme due to the formation of nitrogen. The suspension was then cooled to 0°C, filtered and washed with xylene to provide the title compound.

### Step 5 Ethyl 4-(methylthio)-6-oxo-6,7,8,9-tetrahydropyrido[3,2-b]indolizine-7-carboxylate

To a solution of the compound of Step 4 (0.35 g, 1.6 mmol) in DMF (20 mL) at 0°C was added NaH (1.2 eq.). After a period of 5 min., nBu₄NI (0.10 g) and ethyl 4-bromobutyrate (0.40 mL). were added. After a period of 1 h at room temperature, the reaction mixture was poured over saturated NH₄Cl and EtOAc. The organic phase was separated, washed with H₂O and dried over NaSO₄. After evaporation the crude product was purified by flash chromatography. The bis ester was then dissolved in THF (7.0 mL) and a 1.06 M of THF solution of potassium tert-butoxide (2.2 mL) was added at 0°C. After a period of 1 h at room temperature, the reaction mixture was then poured over saturated NH₄Cl and EtOAc. The organic phase was separated, dried over Na₂SO₄ and evaporated under reduced pressure to provide the title compound as a mixture of ethyl and methyl ester.

### Step 6 4-(Methylthio)-8,9-dihydropyrido[3,2-b]indolizin-6(7H)-one

To the compound of Step 5, (0.32 g) were added EtOH (8.0 mL) and concentrated HCl (2.0 mL). The resulting suspension was refluxed for 5 h. The reaction mixture was partitioned between EtOAc and Na₂CO₃. The organic phase was separated and evaporated to provide the title compound.

### Step 7 Ethyl (2E, 2Z)-[4-(methylthio)-8,9-dihydropyrido[3,2-b]indolizin-6(7H)-ylidene]ethanoate

To a DMF solution (12 mL) of triethyl phosphonoacetate (0.45 g, 2.17 mmol) were added 80% NaH (0.06 g, 2.00 mmol) and the compound of Step 6 (0.22 g, 1.00 mmole). After a period of 4 h at 55°C, the reaction mixture was poured over saturated NH₄Cl and EtOAc. The organic phase was separated and evaporated under reduced pressure. The crude product was purified by flash chromatography to afford the title compound.

### Step 8 Ethyl [4-(methylthio)-6,7,8,9-tetrahydropyrido[3,2-b]indolizin-6-yl]acetate

The compound of Step 7 was dissolved in MeOH - THF using heat for dissolution. To the previous cooled solution was added at room temperature PtO₂ and the resulting mixture was maintained for 18 h under an atmospheric pressure of hydrogen. The reaction mixture was filtered carefully over Celite using CH₂Cl₂. The filtrate was evaporated under reduced pressure to provide the title compound. Alternatively, the compound of Step 7 can be hydrogenated with Pd (OH)₂ in EtOAc at 40 PSI of H₂ for 18h.

### Step 9 Ethyl [4-(methylsulfonyl)-6,7,8,9-tetrahydropyrido[3,2-b]indolizin-6-yl]acetate

To the compound of Step 8 (0.08 g, 0.27 mmol) in MeOH (3.0 mL) were added Na₂WO₄ (0.10 g) and 30% H₂O₂ (600 µL). After a period of 1 h, the reaction mixture was partitioned between H₂O and EtOAc. The organic phase was washed with H₂O, separated and evaporated. The title compound was purified by flash chromatography.

### Step 10 Ethyl [5-[(4-chlorophenyl)thio]-4-(methylsulfonyl)-6,7,8,9-tetrahydropyrido[3,2-b]indolizin-6-yl]acetate

To a 1,2-dichloroethane solution (2.0 mL) of 4,4'-dichlorodiphenyl disulfide (0.24 g) was added SO₂Cl₂ (50 µL). To the compound of Step 9 (0.05 g) in DMF (2.0 mL) was added the previous mixture (≈ 180 µL). The reaction was followed by ¹H NMR and maintained at room temperature until no starting material remained. The reaction mixture was poured over saturated NaHCO₃ and EtOAc. The organic phase was separated, evaporated and the title compound purified by flash chromatography.

### Step 11 [5-[(4-Chlorophenyl)thio]-4-(methylsulfonyl)-6,7,8,9-tetrahydropyrido [3,2-b]indolizin-6-yl]acetic acid

To the compound of Step 10 dissolved in a 1/1 mixture of THF-MeOH was added 1N NaOH. After a period of 18 h at room temperature, the reaction mixture was partitioned between saturated NH₄Cl and EtOAc. The organic phase was separated, dried over Na₂SO₄ and evaporated to provide the title compound.
¹H NMR (500 MHz, acetone-d₆) δ 11.00 (bs, 1H), 8.60 (d, 1H), 7.80 (d, 1H), 7.20 (d, 2H), 7.00 (d, 2H), 4.65 (m, 1H), 4.20 (m, 1H), 3.75 (m, 1H), 3.35 (s, 3H), 2.80 to 2.10 (m, 6H).

### EXAMPLE 2

### [5-[(4-Chlorophenyl)thio]-4-(methylthio)-6,7,8,9-tetrahydropyrido[3,2-b]indolizin-6-yl]acetic acid (Compound H)

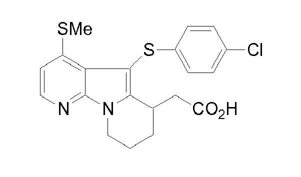

The title compound can be prepared from the compound of Example 1, Step 8 in a similar manner as described in Example 1, Step 10 and 11.
m/z 418.

### EXAMPLE 3

### [5-[(3,4-Dichlorophenyl)thio]-4-(methylsulfonyl)-6,7,8,9-tetrahydropyrido[3,2-b] indolizin-6-yl]acetic acid (Compound I)

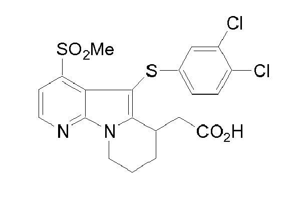

The title compound was prepared as described in Example 1 using bis(3,4-dichlorophenyl)disulfide in Step 10.
¹H NMR (500 MHz, acetone-d₆) δ 8.55 (d, 1H), 7.85 (d, 1H), 7.35 (d, 1H), 7.15 (s, 1H), 6.95 (d, 1H), 4.60 (m, 1H), 4.15 (m, 1H), 3.80 (m, 1H), 3.40 (s, 3H), 2.80 to 2.10 (m, 6H). m/z 484.

The enantiomers were separated on a Chiralecel OD column 25 cm x 20 mm using 30 % isopropanol 17 % ethanol 0.2 % acetic acid in hexane, flow rate 8 ml/min. Their pureties were verified on a Chiralecel OD column 25 cm x 4.6 mm using 35 % isopropanol 0.2 % acetic acid in hexane, flow rate 1.0 ml/min. More mobile enantiomer Tr = 9.7 min, less mobile enantiomer Tr 11.1 min.

### EXAMPLE 4

### [5-(4-Chlorobenzoyl)-4-(methylsulfonyl)-6,7,8,9-tetrahydropyrido[3,2-b]indolizin-6-yl]acetic acid (Compound J)

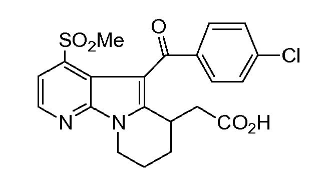

### Step 1 Ethyl [5-(4-chlorobenzoyl)-4-(methylthio)-6,7,8,9-tetrahydropyrido[3,2-b]indolizin-6-yl]acetate

To a solution of 4-chlorobenzoyl chloride (0.30 g, 1.7 mmol) in 1,2-dichloethane (6.0 mL) was added AlCl₃ (0.24 g, 1.8 mmole). After a period of 5 min. a solution of ethyl [4-(methylthio)-6,7,8,9-tetrahydropyrido[3,2-b] indolizin-6-yl]acetate from Example 1 Step 8 (0.15 g, 0.47 mmole) in 1,2-dichloroethane (6.0 mL) was added to the previous mixture. After a period of 4h, at 80°C, the reaction mixture was partitioned between EtOAc and NaHCO₃. The organic phase was separated, dried over Na₂SO₄ and evaporated. The title compound was purified by flash chromatography.

### Step 2 Ethyl [5-(4-chlorobenzoyl)-4-(methylsulfonyl)-6,7,8,9-tetrahydropyrido [3,2-b]indolizin-6-yl]acetate

To a solution of ethyl[5-(4-chlorobenzoyl)-4-(methylthio)-6,7,8-9-tetrahydropyrido[3,2-b]indolizin-6yl] acetate (0.12 g, 0.27 mmole) in MeOH (5.0 mL) were added Na₂WO₄ (0.1 g) and 30% H₂O₂ (300 µL). The reaction mixture was stirred at 55°C for 1h. The reaction mixture was then partitioned between H₂O and EtOAc. The organic phase was washed with H₂O, dried over Na₂SO₄ and evaporated. The title compound was purified by flash chromatography.

### Step 3 [5-(4-Chlorobenzoyl)-4-(methylsulfonyl)-6,7,8,9-tetrahydropyrido[3,2-b]indolizin-6-yl]acetic acid

Ethyl [5-(4-chlorobenzoyl)-4-(methylsulfonyl)-6,7-8,9-tetrahydropyrido[3,2-b]indolizin-6yl]acetate was treated as described in Example 1 Step 11 to provide the title compound.
¹H NMR (500 MHz, acetone-d₆) δ 8.55 (d, 1H), 7.90 (d, 2H), 7.65 (d, 1H), 7.45 (d, 2H), 4.55 (m, 1H), 4.25 (m, 1H), 3.45 (m, 1H), 3.20 (s, 3H), 2.05 to 3.00 (m, 6H).
m/z 446.

### EXAMPLE 5

### [5-(4-Bromophenyl)thio]-4-(methylsulfonyl)-6,7,8,9-tetrahydropyrido[3,2-b]indolizin-6-yl]acetic acid (Compound K)

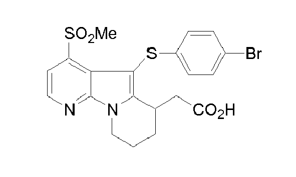

The title compound was prepared as described in Example 1 using 4,4'-dibromodiphenyl disulfide.
¹H NMR (500 MHz, Acetone-d6) δ 8.60 (d, 1H), 7.80 (d, 1H), 7.35 (d, 2H), 7.00 (d, 2H), 4.65 (m, 1H), 4.20 (m, 1H), 3.80 (m, 1H), 3.35 (s, 3H), 2.80 to 2.10 (m, 6H).

### EXAMPLE 6 METHOD-1

### [9-[(3,4-Dichlorophenyl)thio]-1-(methylsulfonyl)-7,8-dihydro-6H-pyrido[3,4-b]pyrrolizin-8-yl]acetic acid (Compound L)

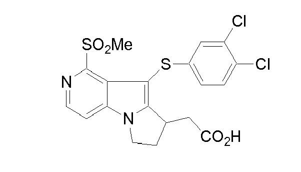

### Step 1 2-(Methylthio)nicotinaldehyde

The title compound was prepared from 2-bromonicotinaldehyde (A. Numata Synthesis 1999 p.306) as described in Example 1 Step 2 except the solution was heated at 55°C for 2 hr.

### Step 2 Methyl (2Z)-2-azido-3-[2-(methylthio)pyridin-3-yl]prop-2-enoate

The title compound was prepared as described in Example 1 Step 3.

### Step 3 Methyl 4-(methylthio)-1H-pyrrolo[3,2-c]pyridine-2-carboxylate

A solution of methyl (2Z)-2-azido-3-[2-(methylthio)pyridin-3-yl]prop-2-enoate (1.00 g, 4.00 mmol) in mesitylene (50 mL) was heated at 160°C for a period of 1 h. The reaction mixture was cooled to room temperature then to 0°C , the precipitate was filtered and washed with cold mesitylene to provide the title compound.

### Step 4 Methyl 1-(methylthio)-8-oxo-7,8-dihydro-6H-pyrido[3,4-b]pyrrolizine-7-carboxylate

To a suspension of methyl 4-(methylthio)-1*H*-pyrrolo[3,2-c]pyridine-2-carboxylate (0.30 g, 1.35 mmol) in THF (3 mL)- toluene (12.0 mL) were added a 1.06 M THF solution of potassium tert-butoxide (1.42 mL / 1.41 mmol)and methyl acrylate (300 µL). The resulting mixture was heated at 80°C for 18h. The mixture was partitioned between EtOAc and NH₄Cl, and filtered through Celite. The organic phase was separated, dried over Na₂SO₄ and filtered, to provide the title compound.

### Step 5 1-(Methylthio)-6,7-dihydro-8H-pyrido[3,4-b]pyrrolizin-8-one

Methyl 1-(methylthio)-8-oxo-7,8-dihydro-6*H*-pyrido[3,4-b] pyrrolizine-7-carboxylate was converted to the title compound as described in Example 1 Step 6.

### Step 6 Methyl [8-hydroxy-1-(methylthio)-7,8-dihydro-6H-pyrido[3,4-b]pyrrolizin-8-yl]acetate

A mixture of 1-(methylthio)-6,7-dihydro-8*H*-pyrido[3,4-b]pyrrolizin-8-one (0.15 g, 0.68 mmol), methyl bromoacetate (0.34 mL), Zn-Cu (0.226 g) in THF (3.0 mL) was sonicated for 2 h. The mixture was then heated at 60°C for 5 min. until completion of the reaction. The reaction mixture was partitioned between EtOAc and NH₄Cl. The organic phase was separated, dried over Na₂SO₄, filtered and evaporated under reduced pressure to provide the title compound. The compound was purified by flash chromatography.

### Step 7 Methyl [1-(methylthio)-7,8-dihydro-6H-pyrido[3,4-b]pyrrolizin-8-yl]acetate

To NaI (0.300 g) in CH₃CN (3.2 mL) was added TMSCl (0.266 mL). This mixture was added to a suspension of methyl [8-hydroxy-1-(methylthio)-7,8-dihydro-6*H*-pyrido[3,4-b]pyrrolizin-8-yl] acetate (0.15 g, 0.515 mmol) in CH₃CN (1.5 mL), in a water bath. After a period of 0.5 h, the reaction mixture was partitioned between EtOAc and NaHCO₃. The organic phase was separated, washed with sodium thiosulphate, dried over MgSO₄ and evaporated. The title compound was purified by flash chromatography.

### Step 8 Methyl [1-(methylsulfonyl)-7,8-dihydro-6H-pyrido[3,4-b]pyrrolizin-8-yl]acetate

Methyl [1-(methylthio)-7,8-dihydro-6*H*-pyrido[3,4-b]pyrrolizin-8-yl]acetate was converted to the title compound as described in Example 1 Step 9.

### Step 9 [9-[(3,4-Dichlorophenyl)thio]-1-(methylsulfonyl)-7,8-dihydro-6H-pyrido[3,4-b]pyrrolizin-8-yL]acetic acid

Methyl [1-(methylsulfonyl)-7,8-dihydro-6*H*-pyrido[3,4-b]pyrrolizin-8-yl]acetate was converted to the title compound as described in Example 1, Steps 10 and 11, using bis (3,4-dichlorophenyl)disulfide in Step 10.
¹H NMR (500 MHz, acetone-d₆) δ 8.35 (d, 1H) 7.80 (d, 1H), 7. 35 (d, 1H), 7.15 (s, 1H), 6.95 (d, 1H), 4.55 (m, 1H), 4.35 (m, 1H), 3.90 (m, 1H), 3.30 (s, 3H), 3.15 (m, 1H), 3.05 (m, 1H), 2.80 (m, 1H), 2.50 (m, 1H).

### EXAMPLE 6 METHOD-2

### [9-[(3,4-Dichlorophenyl)thio]-1-(methylsulfonyl)-7,8-dihydro-6H-pyrido[3,4-b]pyrrolizin-8-yl]acetic acid

### Step 1 1-(Methylthio)-7,8-dihydro-6H-pyrido[3,4-b]pyrrolizin-8-ol

To a suspension of 1-(methylthio)-6,7-dihydro-8H-pyrido[3,4-b]pyrrolizin-8-one from Example 6, Method-1 Step 5 (0.55 g, 2.2 mmol) in EtOH (10 mL)-THF (1 mL) was added NaBH₄ (0.10 g, 2.6 mmol) at 0°C. After a period of 30 min. at room temperature, the reaction was quenched by the addition of acetone. The solvents were evaporated under reduced pressure and EtOAC and H₂O were added to the residue. The organic phase was separated, dried over MgSO₄ and evaporated. The title compound was washed with EtOAc/Hexane and filtered.

### Step 2 Dimethyl 2-[1-(methylthio)-7,8-dihydro-6H-pyrido[3.4-b]pyrrolizin-8-yl]malonate

To a suspension of 1-(methylthio)-7,8-dihydro-6H-pyrido[3,4-b]pyrrolizin-8-ol (0.54 g, 2.1 mmol) in THF (10 mL) at -78°C were added 1M NaHMDS in THF (2.35 mL, 2.4 mmol) and diphenyl chlorophosphate (0.53 mL, 2.6 mmol). After a period of 30 min. dimethyl malonate (0.73 mL, 6.4 mmol) and 1M NaHMDS in THF (6.8 mL, 6.8 mmol) were added. The reaction mixture was brought to 0°C and then to room temperature. The mixture was then partitioned between ETOAc and NH₄Cl. The organic phase was dried over MgSO₄, filtered and evaporated. The title compound was purified by flash chromatography.

### Step 3 Methyl [1-(methylthio)-7,8-dihydro-6H-pyrido[3,4-b]pyrrolizin-8-yl]-acetate

To a mixture of dimethyl 2-[1-(methylthio)-7,8-dihydro-6H-pyrido[3,4-b]pyrrolizin-8-yl]malonate (0.59 g, 2.17 mmol) and DMSO (4mL) was added NaCl (0.45 g) in H2O (0.45 mL). After a period of 18 h at 150°C, the reaction mixture was partitioned between ETOAc and H2O. The organic phase was separated, dried over Na2SO4 and evaporated. The title compound was then purified by flash chromatography.

### Step 4 [9-[(3,4-Dichlorophenyl)thio]-1-(methylsulfonyl)-7,8-dihydro-6H-pyrido[3,4-b]pyrrolizin-8-yl]acetic acid

The title compound was obtained from methyl [1-(methylthio)-7,8-dihydro-6H-pyrido[3,4-b]pyrrolizin-8yl]acetate as described in Example 6, Method-1, Steps 8 to 9.

### EXAMPLE 7

### [10-[(3,4-Dichlorophenyl)sulfanyl]-1-(methylsulfonyl)-6,7,8,9-tetrahydropyrido[3,4-b]indolizin-9-yl]acetic acid (Compound M)

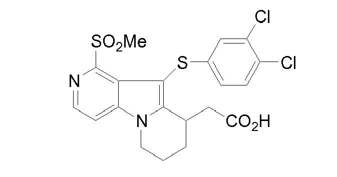

### Step 1 Ethyl [1-(methylsulfonyl)-6,7,8,9-tetrahydropyrido[3,4-b]indolizin-9-yl]acetate

The title compound was prepared from the product of Example 6, Step 3 in the same manner as described in Example 1, Steps 5 to 9.

### Step 2 [10-[(3,4-Dichlorophenyl)sulfanyl]-1-(methylsulfonyl)-6,7,8,9-tetrahydropyrido[3,4-b]indolizin-9-yl]acetic acid

The product of Step 1 was converted to the title compound in the same manner as Example 1, Steps 10-11, using bis (3,4-dichlorophenyl)disulfide in Step 10.
MS M+1=485.

### EXAMPLE 8

### (4-(Methylsulfonyl)-5-{[4-(trifluoromethyl)phenyl]thio}-6,7,8,9-tetrahydropyrido[3,2-b]indolizin-6-yl)acetic acid (Compound N)

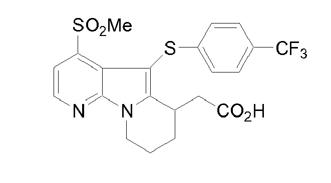

The title compound was prepared as described in Example 1 using bis[4-trifluoromethyl)phenyl]disulfide.
¹H NMR (500 MHz, acetone-d₆) δ 8.55 (d, 1H), 7.75 (d, 1H), 7.45 (d, 2H), 7.15 (d, 2H), 4.55 (m, 1H), 4.15 (m, 1H), 3.80 (m, 1H), 3.30 (s, 3H), 2.80 to 2.10 (m, 6H).
m/z 513 (M+1).

### EXAMPLE 9

### [5-[(2-Chloro-4-fluorophenyl)thio]-4-(methylsulfonyl)-6,7,8,9-tetrahydropyrido[3,2-b]indolizin-6-yl]acetic acid (Compound O)

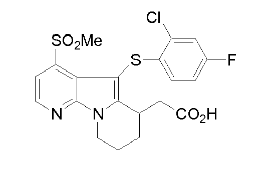

The title compound was prepared as described in Example 1 using bis(2-chloro-4-fluorophenyl)disulfide.
m/z 469 (M+1).

### EXAMPLE 10

### [4-(Methylsulfonyl)-5-(2-naphthylthio)-6,7,8,9-tetrahydropyrido[3,2-b]indolizin-6-yl]acetic acid (Compound P)

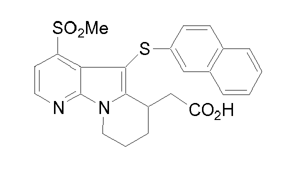

The title compound was prepared as described in Example 1 using di(2-naphthyl) disulfide.
M/z 467 (M+1).

### EXAMPLE 11

### [5-[(2,3-Dichlorophenyl)thio]-4-(methylsulfonyl)-6,7,8,9-tetrahydropyrido[3,2-b]indolizin-6-yl]acetic acid (Compound Q)

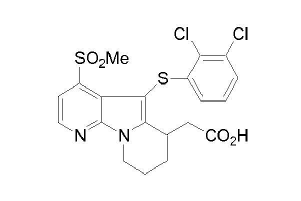

The title compound was prepared as described in Example 1 using bis(2,3-dichlorophenyl)disulfide.
¹H NMR (500 MHz, acetone-d₆) δ 8.85 (d, 1H), 7.80 (d, 1H), 7.30 (d, 1H), 7.00 (t, 1H), 6.60 (d, 1H), 4.60 (m, 1H), 4.20 (m, 1H), 3.80 (m, 1H), 3.40 (s, 3H), 2.80 to 2.10 (m, 6H).

### EXAMPLE 12

### [5-[(4-Methylphenyl)thio]-4-(methylsulfonyl)-6,7,8,9-tetrahydropyrido[3,2-b]indolizin-6-yl]acetic acid (Compound R)

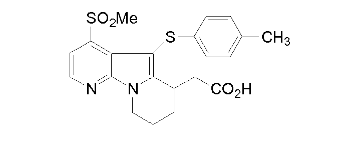

The title compound was prepared as described in Example 1 using p-tolyl disulfide.
¹H NMR (500 MHz, acetone-d₆) δ 8.55 (d, 1H), 7.80 (d, 1H), 6.95 (m, 4H), 4.60 (m, 1H), 4.15 (m, 1H), 3.80 (m,1H), 3.35 (s, 3H), 2.80 to 2.10 (m, 6H).

### EXAMPLE 13

### [4-(Methylsulfonyl)-5-(phenylthio)-6,7,8,9-tetrahydropyrido[3,2-b]indolizin-6-yl]acetic acid (Compound S)

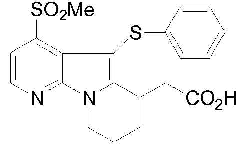

The title compound was prepared as described in Example 1 using diphenyl disulfide.
¹H NMR (500 MHz, acetone-d₆) δ 8.55 (d, 1H), 7.80 (d, 1H), 7.15 to 6.90 (m, 5H), 4.60 (m, 1H), 4.15 (m, 1H), 3.75 (m, 1H), 3.30 (s, 3H), 2.80 to 2.10 (m, 6H).

### EXAMPLE 14

### [5-[(2,4-Dichlorophenyl)thio]-4-(methylsulfonyl)-6,7,8,9-tetrahydropyrido[3,2-b]indolizin-6-yl]acetic acid (Compound T)

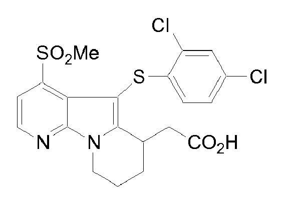

The title compound was prepared as described in Example 1 using bis(2,4-dichlorophenyl)disulfide. The disulfide was prepared from 2,4-dichlorothiophenyl using Br₂ in ether.
¹H NMR (500 MHz, acetone-d₆) δ 8.55 (d,1H), 7.85 (d, 1H), 7.35 (s, 1H), 7.00 (d, 1H), 6.65 (d, 1H), 4.55 (m, 1H), 4.15 (m, 1H), 3.80 (m, 1H), 3.35 (s, 3H), 2.80 to 2.10 (m, 6H).

### EXAMPLE 15

### [5-[(4-Chlorophenyl)thio]-4-(methylsulfonyl)-6,7,8,9-tetrahydropyrido[4,3-b]indolizin-6-yl]acetic acid (Compound U)

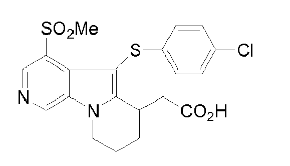

The title compound was prepared as described in Example 1 from 3-chloronicotinaldehyde (Heterocycles p. 151, 1993) except the terminal cyclization was performed by adding the azide to decalin at reflux.
¹H NMR (500 MHz, acetone-d₆) δ 9.20 (s, 1H), 8.85 (s, 1H), 7.20 (d, 2H), 7.00 (d, 2H), 4.70 (m, 1H), 4.30 (m, 1H), 3.75 (m, 1H), 3.35 (s, 3H), 2.80 to 2.10 (m, 6H).

### EXAMPLE 16

### [9-[(4-Chlorophenyl)thio]-1-(methylsulfonyl)-7,8-dihydro-6H-pyrido[3,4-b]pyrrolizin-8-yl]acetic acid (Compound V)

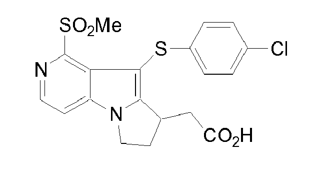

The title compound was prepared from the product of Example 6 Method 1 Step 8, as described in the procedures outlined in Example 1 Steps 10 and 11, using bis (4-chlorophenyl)disulfide in Step 10.
¹H NMR (500 MHz, acetone-d₆) δ 8.25-8.3 (m, 1H), 7.71-7.75 (m, 1H), 7.12-7.17 (m, 2H), 6.97-7.04 (m, 2H), 4.45-4.51 (m, 1H), 4.32-4.39 (m, 1H), 3.73-3.80 (m, 1H), 3.29 (s, 3H), 3.15-3.21 (m, 1H), 2.99-3.08 (m, 1H), 2.66-2.73 (m, 1H), 2.46-2.54 (m, 1H).

### EXAMPLE 17

### (-)-[(4-Chlorobenzyl)-7-fluoro-5-methanesulfonyl)-1,2,3,4-tetrahydrocyclopenta[b]indol-3-yl]acetic acid (Compound E)

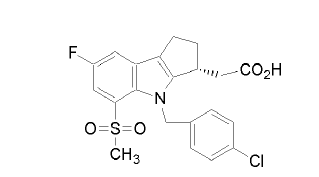

### Step 1: (+/-)-(7-Fluoro-1,2,3,4-tetrahydrocyclopenta[b]indol-3-yl)acetic acid ethyl ester.

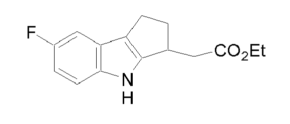

A solution of 10.00 g of 4-fluoro-2-iodoaniline, 6.57 g of ethyl 2-(2-oxocyclopentyl)acetate and 121 mg of p-toluenesulfonic acid in 100 ml of benzene was refluxed with a Dean-Stark trap under a N₂ atmosphere for 24h. After this time, the benzene was removed under distillation. Then, 60ml of DMF was added and the solution was degassed before 19 ml of Hunig's base followed by 405 mg of Pd(OAc)₂ were added successively. The solution was heated to 115°C for 3 h, then cooled to room temperature. To quench the reaction, 300 ml of 1 N HCl and 200 ml of ethyl acetate were added and the mixture was filtered through Celite. The phases were separated and the acidic phase was extracted twice with 200 ml of ethyl acetate. The organic layers were combined, washed with brine, dried over anhydrous Na₂SO₄, filtered through Celite and concentrated. The crude material was further purified by flash chromatography eluting with 100% toluene, to provide the title compound.
¹H NMR (acetone-d₆) δ 9.76 (br s, 1H), 7.34 (dd, 1H), 7.03 (d, 1H), 6.78 (td, 1H), 4.14 (q, 2H), 3.57 (m, 1H), 2.85-2.55 (m, 5H), 2.15 (m,1H), 1.22 (t, 3H).

### Step 2: (+/-)-(7-Fluoro-1,2,3,4-tetrahydrocyclopenta[b]indol-3-yl)acetic acid

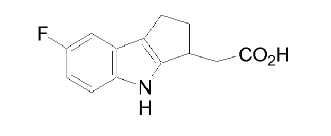

To a solution of 1.24 g of the ester from Step 1 in 14 mL of tetrahydrofuran (THF) at room temperature, 7 mL of MeOH followed by 7 mL of 2N NaOH were added. After 2.5 h, the reaction mixture was poured into a separatory funnel containing ethyl acetate (EtOAc)/1N HCl. The phases were separated and the acidic phase was extracted twice with EtOAc. The organic layers were combined, washed with brine, dried over anhydrous Na₂SO₄ and evaporated to dryness to yield a crude oil that was used as such in the next step (>90% purity).
¹H NMR (acetone-d₆) δ 10.90 (br s, 1H), 9.77 (br s, 1H), 7.34 (dd, 1H), 7.04 (dd, 1H), 6.79 (td, 1H), 3.56 (m, 1H), 2.90-2.50 (m, 5H), 2.16 (m, 1H). MS (-APCI) m/z 232.2 (M-H)⁻.

### Step 3: (+/-)-(5-bromo-7-fluoro-1,2,3,4-tetrahydrocyclopenta[b]indol-3-yl)acetic acid

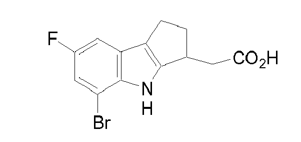

To a solution of 2.20 g of the acid from Step 2 (>90% purity) in 30 mL of pyridine, 6.85 g of pyridinium tribromide (90% purity) was added at -40°C. The suspension was stirred for 10 min at 0°C and warmed to room temperature for 30 min. Then, the solvent was removed without heating under high vacuum. The crude material was dissolved in 40 mL of AcOH and 2.88 g of Zn dust was added portion wise to the cold solution at 0°C. The suspension was stirred for 15 min at 15°C and warmed to room temperature for an additional 15 min. At this time, the reaction mixture was quenched by the addition of 1N HCl and this mixture was poured into a separatory funnel containing brine/EtOAc. The layers were separated and the organic layer was washed with water, brine, dried over anhydrous Na₂SO₄ and concentrated. This material was used without further purification in the next step.
¹H NMR (acetone-d₆) δ 10.77 (br s, 1H), 9.84 (br s, 1H), 7.09 (m, 2H), 3.60 (m, 1H), 2.95-2.65 (m, 4H), 2.56 (dd, 1H), 2.19 (m, 1H).

### Step 4: (+/-)-[5-bromo-4-(4-chlorobenyl)-7-fluoro-1,2,3,4-tetrahdrocyclopenta[b]indol-3-yl]-acetic acid

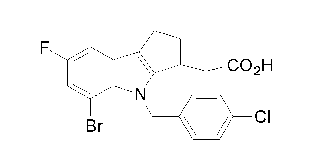

To a solution of 2.13 g of the acid from Step 3 in 10 mL of THF, a solution of diazomethane in ether was added in excess until complete consumption of the acid as monitored on TLC. Then, the solvents were removed under vacuum. To a solution of the crude methyl ester thus formed in 20 mL of DMF, 539 mg of a NaH suspension (60% in oil) was added at -78°C. The suspension was stirred for 10 min at 0°C, cooled again to -78°C and treated with 1.70 g of 4-chlorobenzyl bromide. After 5 min, the temperature was warmed to 0°C and the mixture was stirred for 20 min. At this time, the reaction was quenched by the addition of 2 mL of AcOH and this mixture was poured into a separatory funnel containing 1N HCl/EtOAc. The layers were separated and the organic layer was washed with brine, dried over anhydrous Na₂SO₄ and concentrated. The alkylated material was hydrolyzed using the procedure described in Step 2. The crude material was further purified by trituration with EtOAc/hexanes to provide the title compound.
¹H NMR (acetone-d₆) δ 10.70 (br s, 1H), 7.31 (d, 2H), 7.18 (d, 1H), 7.06 (d, 1H), 6.92 (d, 2H), 5.90 (d, 1H), 5.74 (d, 1H), 3.61 (m, 1H), 3.00-2.70 (m, 3H), 2.65 (dd, 1H), 2.39 (dd, 1H), 2.26 (m, 1H). MS (-APCI) m/z 436.3, 434.5 (M-H)⁻.

### Step 5: (+)-[5-bromo-4-(4-chlorobenzyl)-7-fluoro-1,2,3,4-tetrahydrocyclopenta[b]indol-3-yl}acetic acid

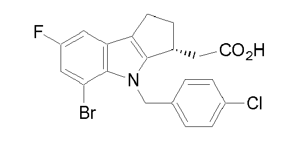

To a solution of 2.35 g of the acid of Step 4 in 130 mL of EtOH at 80°C, was added 780 µL of (S)-(-)-1-(1-naphthyl)ethylamine. The solution was cooled to room temperature and stirred overnight. The salt recovered (1.7 g) was recrystallized again with 200 mL of EtOH. After filtration, the white solid salt obtained was neutralized with 1N HCl and the product was extracted with EtOAc. The organic layer was washed with brine, dried over anhydrous Na₂SO₄ and concentrated. The material was filtered over a pad of SiO₂ by eluting with EtOAc to produce the title enantiomer. Retention times of the two enantiomers were respectively 7.5 min and 9.4 min [ChiralPak AD column, hexane/2-propanol/acetic acid (95:5:0.1)]. The more polar enantiomer was in 98% ee.
ee = 98%; Retention time = 9.4 min [ChiralPak AD column: 250 x 4.6 mm, hexanes/2-propanol/acetic acid (75:25:0.1)]; [α]_{D}²¹ = +39.2° (c 1.0, MeOH).

### Step 6: (-)-[4-(4-chlorobenzyl)-7-fluoro-5-(methanesulfonyl)-1,2,3,4-tetrahydrocyclopenta[b]-indol-3-yl}acetic acid and sodium salt

The acid from Step 5 (15.4 g) was first esterified with diazomethane. The sulfonylation was accomplished by mixing the ester thus formed with 16.3 g of methanesulfinic acid sodium salt and 30.2 g of CuI (I) in N-methylpyrrolidinone. The suspension was degassed under a flow of N₂, heated to 150°C and stirred for 3h, then cooled to room temperature. To quench the reaction, 500 ml of ethyl acetate and 500 ml of hexanes were added and the mixture was filtered through a pad of SiO₂ by eluting with EtOAc. The organic phases were concentrated. The crude oil was dissolved with EtOAc, washed three times with water one time with brine, dried over anhydrous Na₂SO₄, filtered and concentrated. The crude material was further purified by flash chromatography eluting with a gradient from 100% toluene to 50% toluene in EtOAc, to provide 14 g of the sulfonated ester, which was hydrolyzed using the procedure described in Step 2. The title compound was obtained after two successive recrystallizations: isopropyl acetate / heptane followed by CH₂Cl₂ / hexanes.
¹H NMR (500 MHz acetone-d₆) δ 10.73 (br s, 1H), 7.57 (d, 2H, *J*=8.8 Hz), 7.31 (m, 1H), 7.29 (m, 1H), 6.84 (d, 2H, *J*=8.8 Hz), 6.29 (d, 1H, *J_{AB}*=17.8 Hz), 5.79 (d, 1H, *J_{AB}*=17.8 Hz), 3.43 (m, 1H), 2.98 (s, 3H), 2.94 (m, 1H), 2.85-2.65 (m, 3H), 2.42 (dd, 1H, *J₁*=16.1 Hz, *J₂*=10.3 Hz), 2.27 (m, 1H). ¹³C NMR (125 MHz acetone-d₆) δ 173.0, 156.5 (d, *J*_{CF}=237 Hz), 153.9, 139.2, 133.7, 133.3, 130.0 (d, *J*_{CF}=8.9 Hz), 129.6, 128.2, 127.5 (d, *J*_{CF}=7.6 Hz), 122.2 (d, *J*_{CF}=4.2 Hz), 112.3 (d, *J*_{CF}=29.4 Hz), 111.0 (d, *J*_{CF}=22.6 Hz), 50.8, 44.7, 38.6, 36.6, 36.5, 23.3. MS (-APCI) m/z 436.1, 434.1 (M-H)⁻.
ee = 97%; Retention time = 15.3 min [ChiralCel OD column: 250 x 4.6 mm, hexanes/2-propanol/ethanol/acetic acid (90:5:5:0.2)]; [α]_{D}²¹ = -29.3° (c 1.0, MeOH). Mp 175.0°C.

The sodium salt was prepared by the treatment of 6.45 g (14.80 mmol) of the above acid compound in EtOH (100 mL) with 14.80 mL of an aqueous 1N NaOH solution. The organic solvent was removed under vacuum and the crude solid was dissolved in 1.2L of isopropyl alcohol under reflux. The final volume was reduced to 500 mL by distillation of the solvent. The sodium salt crystallized by cooling to rt. The crystalline sodium salt was suspended in H₂O, frozen with a dry ice bath and lyophilized under high vacuum to give the title compound as the sodium salt.
¹H NMR (500 MHz DMSO-d₆) δ 7.63 (dd, 1H, *J₁*=8.5 Hz, *J₂*=2.6 Hz), 7.47 (dd, 1H, *J₁*=9.7 Hz, *J₂*=2.6 Hz), 7.33 (d, 2H, *J*=8.4 Hz), 6.70 (d, 2H, *J*=8.4 Hz), 6.06 (d, 1H, *J_{AB}*=17.9 Hz), 5.76 (d, 1H, *J_{AB}*=17.9 Hz), 3.29 (m, 1H), 3.08 (s, 3H), 2.80 (m, 1H), 2.69 (m, 1H), 2.55 (m, 1H), 2.18 (m, 2H), 1.93 (dd, 1H, *J₁*=14.4 Hz, *J₂*=9.7 Hz).

### EXAMPLE 17A

### Alternative procedure for (+/-)- [5-bromo-4-(4-chlorobenzyl)-7-fluoro-1,2,3,4-tetrahydrocyclopenta[b]indol-3-yl]acetic acid (Example 17, Step 4)

### Step 1: (+/-)-7-fluoro-1,2,3,4-tetrahydrocyclopenta[b]indol-3-yl)acetic acid dicyclohexylamine (DCHA) salt

A 0.526 M solution of 2-bromo-4-fluoroaniline in xylene along with ethyl (2-oxocyclopentyl) acetate (1.5 eq) and sulfuric acid (0.02 eq) was heated to reflux for 20 hours. Water was azeotropically removed with a Dean-Stark apparatus. The reaction was followed by NMR and after 20 hours, an 80-85% conversion to the desired imine intermediate was generally observed. The reaction mixture was washed with 1M sodium bicarbonate (0.2 volumes) for 15 minutes and the organic fraction was evaporated. The remaining syrup was distilled under vacuum (0.5 mm Hg). Residual xylenes distilled at 30°C, then excess ketone and unreacted aniline were recovered in the 50-110°C range; the imine was recovered in the 110-180°C fraction as a light brown clear liquid with 83% purity.

The imine intermediate was then added to a degased mixture of potassium acetate (3 eq), tetra-n-butylammonium chloride monohydrate (1 eq), palladium acetate (0.03 eq) and N,N-dimethylacetamide (final concentration of imine = 0.365 M). The reaction mixture was heated to 115°C for 5 hours and allowed to cool to room temperature. 3N KOH (3 eq) was then added and the mixture was stirred at room temperature for 1 hour. The reaction mixture was diluted with water (1.0 volume), washed with toluene (3x0.75 volume). The aqueous phase was acidified to pH 1 with 3N HCl and extracted with tertbutyl methyl ether (2x0.75 volume). The combined organic fractions were washed with water (0.75 volume). To the clear light brown solution was added dicyclohexylamine (1 eq) and the solution was stirred at room temperature for 16 hours. The salt was filtered, washed with ethyl acetate, tertbutyl methyl ether and allowed to dry to give the title compound. Assay: 94 A%.
1H NMR (500 mHz, CDCl3) : δ 9.24 (s, 1H), 7.16-7.08 (m, 2H), 6.82 (t, 1H), 6.2 (br, 2H), 3.6-3.5 (m, 1H), 3.04-2.97 (m, 2H), 2.88-2.70 (m, 3H), 2.66 (dd, 1H), 2.45-2.37 (m, 1H), 2.13-2.05 (m, 2.05), 1.83 (d, 4H), 1.67 (d, 2H), 1.55-1.43 (m, 4H), 1.33-1.11 (m, 6H).

### Step 2: (+/-)-(5-bromo-7-fluoro-1,2,3,4-tetrahydrocyclopenta[b]indol-3-yl)acetic acid

A slurry of the DCHA salt from Step 1 above in dichloromethane (0.241 M solution) was cooled to -20 to -15 °C. Pyridine (2 eq.) was added in one shot and to the slurry was added dropwise bromine (2.5 eq.) over 30 to 45 minutes maintaining the temperature between -20 °C and -15 °C. (At about 1/3 addition of bromine, the reaction mixture was thick and an efficient stirring was needed. Eventually, at about 1/2 addition of bromine, the mixture became "loose" again.) After completion of the addition, the reaction mixture was aged for one additional hour at - 15 °C. Acetic acid (3.04 eq.) was then added over 5 minutes and zinc dust (3.04 eq.) was added portion wise. (A portion of zinc was added at - 15 °C and the mixture was aged for about 5 minutes to ensure that the exotherm was going (about -15 °C to -10 °C)). This operation was repeated with about 5 shots of zinc over about 30 min. When no more exotherm was observed, the remaining zinc was added faster. The whole operation took around 30 to 45 minutes.

After completion of the addition, the batch was warmed to room temperature, aged 1 hour and concentrated. The reaction mixture was switched to methyl t-butyl ether (MTBE, 0.8 volume) and a 10% aqueous acetic acid solution (0.8 volume) was added. The mixture (crystallization of salts, e.g pyridium) was aged at room temperature for 1 hour and filtered through solka-floc. The pad of solka-floc was rinsed with MTBE (ca. 0.2 volume) and the filtrate (biphasic, MTBE/aqueous) was transferred into an extractor. The organic phase was washed with water (0.8 volume). The MTBE extract was concentrated and switched to isopropyl alcohol (IPA, 0.25 volume) to crystallize the compound. Water (0.25 volumes) was added and the batch was aged for 1 hour. Additional water (0.33 volumes) was added over 1 hour. After completion of the water addition, the batch was aged for one additional hour, filtered, and rinse with 30/70 IPA/Water (0.15 volumes). Crystallized bromoacid was dried in the oven at +45 °C.

### Step 3: (+/-)-[5-bromo-4-(4-chlorobenzyl)-7-fluoro-1,2,3,4-tetrahydrocyclopenta[b]indol-3-yl]-acetic acid

The bromoacid of Step 2 was dissolved in dimethylacetamide (0.416 M solution) and cesium carbonate (2.5 eq.) was added in one portion. To the slurry was added in one portion 4-chlorobenzyl chloride (2.5 eq.) and the batch was heated to 50 °C for 20 h. The batch was cooled to r.t. and sodium hydroxide 5N (4.00 eq.) was added over 5 minutes (temperature rose to +40 °C). The reaction was aged at 50 °C for ca. 3 hours, cooled to room temperature and transferred into an L extractor. The solution was diluted with isopropylacetate (IPAc, 2 volumes) and cooled to +15 °C. The solution was acidified with 5N HCl to pH∼2. Layers were separated and the organic layer was washed with water (2x2 volumes). IPAc solution was concentrated and switched to IPA (0.8 volumes) to crystallize the product. Water (8 L) was added over 2 hours and the batch was filtered to give the title compound. The batch can be dried in the oven at +40 °C for 24 hours.

### EXAMPLE 18

### (+/-)-{4-[1-(4-Chlorophenyl)ethyl]-7-fluoro-5-methanesulfonyl-1,2,3,4-tetrahydrocyclopenta[b]indol-3-yl}acetic acid(Compound X)

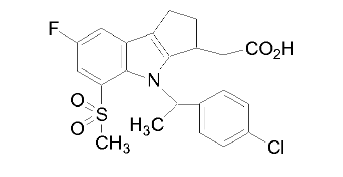

The title compound was synthesized in accordance with the description provided in PCT WO03/062200 published on July 30, 2003.

### EXAMPLE 19

### (+/-)-[9-(4-Chlorobenzyl)-6-fluoro-methanesulfonyl-2,3,4,9-tetrahydro-1H-carbazol-1-yl] acetic acid (Compound Y)

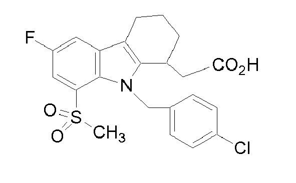

The title compound was synthesized in accordance with the description provided in PCT WO03/062200 published on July 30, 2003.

### EXAMPLE 20

### [4-(4-Chlorobenzyl)-7-fluoro-5-methanesulfonyl-1-oxo-1,2,3,4-tetrahydrocyclopenta[b]indol-3-yl]acetic acid (Compound Z)

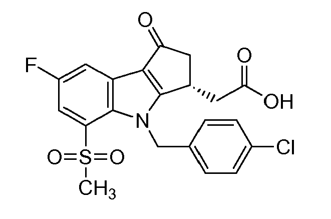

The title compound was synthesized in accordance with the description provided in PCT WO03/062200 published on July 30, 2003.

### EXAMPLE 21

### {9-[(3,4-Dichlorophenyl)thio]-1-isopropyl-7,8-dihydro-6H-pyrido[3,4-b]pyrrolizin-8-yl}acetic acid (Enantiomer A and Enantiomer B) (Compound AA)

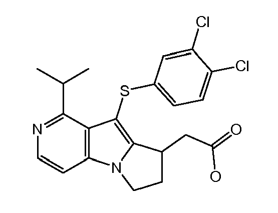

### Step 1 2-Chloronicotinaldehyde

To a solution of diisopropyl amine (110 mL, 780 mmol) in THF (500 mL) was added a 2.5 M hexanes solution of n-BuLi (300 mL, 750 mmol) at -40°C. After 5 min, the reaction mixture was cooled to -95°C then DMPU (15 mL) and 2-chloropyridine (50 mL, 532 mmol) were successively added. The resulting mixture was then warmed and stirred at -78°C for 4h. After this time, the yellow suspension was cooled again to -95°C before DMF (70 mL) was added. The final reaction mixture was warmed to - 78°C and stirred at that temperature for 1.5h. The reaction mixture was poured into cold aqueous HCl (3N, 800 mL) and stirred for 5 min. Aqueous concentrated NH₄OH was added to adjust pH to 7.5. The aqueous layer was extracted three times with EtOAc. The combined organic layer was washed with aqueous NH₄Cl and brine, dried over anhydrous Nₐ2SO₄, filtered and concentrated. The crude material was further purified by a pad of silica gel by eluting with a gradient from 100% hexanes to 100% EtOAc and the product was crystallized in cold hexanes to yield the title compound as a pale yellow solid.

### Step 2 Methyl (2Z)-2-azido-3-(2-chloropyridin-3-yl)prop-2-enoate

A solution of 2-chloronicotinealdehyde (20.0 g, 139.9 mmol) and methyl azidoacetate (32.2 mL, 349.7 mmol) in MeOH (168 mL) was added to a solution of 25% NaOMe in MeOH (80 mL, 349 mmol) at -20 oC. The internal temperature was monitored and maintained at ∼-20 °C during the 30 min. addition. The resulting mixture was then stirred in an ice bath for several hours, followed by overnight in an ice bath in the cold room. The suspension was then poured onto a mixture of ice and NH₄Cl, and the slurry was filtered after 10 min. of stirring. The product was washed with cold H₂O and was then dried under vacuum. The crude material was dissolved in CH₂Cl₂ and MgSO₄ was added. The suspension was filtered through a pad of silica gel, washed with CH₂Cl₂. The filtrate was concentrated under reduced pressure and a beige precipitate (20 g) of the title product was obtained.

### Step 3 Methyl 4-chloro-1H-pyrrolo[3,2-c]pyridine-2-carboxylate

A solution of methyl (2Z)-2-azido-3-[2-chloropyridin-3-yl]prop-2-enoate (21 g, 88 mmol) in mesitylene (880 mL) was heated at reflux for a period of 1 h. The reaction mixture was cooled to room temperature then to 0 °C, and the precipitate was filtered and washed with cold hexane. The material was stirred overnight in 1:20 EtOAc/hexane to give, after filtration, the title product as a pale yellow solid (13.2 g).

### Step 4 Methyl 1-chloro-8-oxo-7,8-dihydro-6H-pyrido[3,4-b]pyrrolizine-7-carboxylate

To a suspension of methyl 4-chloro-1*H*-pyrrolo[3,2-*c*]pyridine-2-carboxylate (12.5 g, 59 mmol) in THF (116 mL) - toluene (460 mL) were added a 1.0 M THF solution of potassium tert-butoxide (64 mL, 64 mmol) and methyl acrylate (55 mL, 611 mmol). The resulting mixture was heated at 100°C for 18h. After this time, the suspension was cooled to room temperature and it was poured into a mixture of saturated aqueous NH₄Cl (400 mL) and hexanes (400 mL). The solids were decanted, filtered and washed with H₂O and hexanes to provide the title compound.

### Step 5 1-Chloro-6,7-dihydro-8H-pyrido[3,4-b]pyrrolizin-8-one

To the compound of the previous step were added isopropanol (8.0 mL) and concentrated HCl (2.0 mL) with heating at 100°C for 1h. The reaction mixture was partitioned between EtOAc and Na₂CO₃. The organic phase was separated, evaporated to provide the title compound.

### Step 6 1-Isopropenyl-6,7-dihydro-8H-pyrido[3,4-b]pyrrolizin-8-one

To a mixture of 1-chloro-6,7-dihydro-8H-pyrido[3,4-b]pyrrolizin-8-one (5.0 g, 24.3 mmol), tris (dibenzylidene acetone)dipalladium (0) (1.0 g, 1.09 mmol) and triphenylarsine (2.70 g, 8.82 mmol) in DMF (100 mL) was added tributylisopropenyl stannane (9.60 g, 29.00 mmol). The resulting mixture was degassed and heated at 78°C for a period of 18 h. The solvent was evaporated under reduced pressure. CH₂Cl₂ and celite were added to the resulting mixture which was then filtered over celite. The title compound was purified by flash chromatography (50% to 100% EtOAc in Hexane).

### Step 7 Ethyl (2E)-(1-isopropenyl-6,7-dihydro-8H-pyrido[3,4-b]pyrrolizin-8-ylidene)ethanoate

To a solution of 1-isopropenyl-6,7-dihydro-8H-pyrido[3,4-b]pyrrolizin-8-one (0.60 g, 2.8 mmol) and triethyl phosphonoacetate (1.00 g, 4.46 mmol) in THF (24 mL) at -78°C was added 80% NaH (0.12 g, 4.00 mmol), the reaction mixture was allowed to warm to 0°C, then to room temperature. The reaction mixture was poured onto saturated NH₄Cl and EtOAc. The organic phase was separated, dried over Na₂SO₄ and evaporated. The title compound was purified by flash chromatography (40% EtOAc in Hexane).

### Step 8 Ethyl (1-isopropyl-7,8-dihydro-6H-pyrido[3,4-b]pyrrolizin-8-yl)acetate

To a solution of ethyl (2E)-(1-isopropenyl-6,7-dihydro-8H-pyrido[3,4-b]pyrrolizin-8-ylidene)ethanoate (0.40 g, 1.4 mmol) in MeOH (20 mL) was added Pd(OH)₂ (0.20 g). The mixture was stirred under 1 atm of H₂ for 3h. The mixture was filtered over celite and evaporated to provide the title compound.

### Step 9 Ethyl {9-[(3,4-dichlorophenyl)thio]-1-isopropyl-7,8-dihydro-6H-pyrido [3,4-b]pyrrolizin-8-yl}acetate

To a solution of bis (3,4-dichlorophenyl)disulfide (0.24 g, 0.67 mmol) in CH₂Cl₂ (5.6 mL) was added SO₂Cl₂ (0.036 mL). The resulting yellow mixture was stirred at room temperature for 1 h. This solution was added to a solution of ethyl (1-isopropyl-7,8-dihydro-6H-pyrido[3,4-b]pyrrolizin-8-yL) acetate (0.15 g, 0.52 mmol) in DMF (5.6 mL) at 0°C. After 1.5 h at 0°C, the reaction mixture was poured over saturated NaHCO₃ and EtOAc. The organic phase was separated, dried over Na₂SO₄, filtered and evaporated. The title compound was purified by flash chromatography (30% to 40% EtOAc in Hexane).

### Step 10 {9-[(3,4-Dichlorophenyl)thio]-1-isopropyl-7,8-dihydro-6H-pyrido[3,4-b]pyrrolizin-8-yl} acetic acid

To a solution of ethyl {9-[(3,4-dichlorophenyl)thio]-1-isopropyl-7,8-dihydro-6H-pyrido[3,4-b]pyrrolizin-8yl} acetate (0.23 g, 0.50 mmol) in THF (5 mL and MeOH (2.5 mL) was added 1.0 M NaOH (1.5 mL, 1.5 mmol). After stirring 18h at RT, HOAc (0.25 mL) was added and the solvent was evaporated. The residue was taken up in EtOAc/H₂O, and the organic layer was washed with H₂O and brine. After drying (Na₂SO₄), the solution was filtered and evaporated. The residue was stirred with 1:1 EtOAc:hex to give, after filtration, the title compound as a white solid.
¹H NMR (MeOH-d₄) δ 1.14-1.26 (m, 6H), 2.47-2.56 (m, 1H), 2.56-2.64 (m, 1H), 2.94-3.05 (m, 2H), 3.81-3.89 (m, 1H), 4.22-4.30 (m, 1H), 4.33-4.44 (m, 2H), 6.93-6.99 (m, 1H), 7.14-7.19 (m, 1H), 7.33-7.39 (m, 1H), 7.54-7.59(m, 1H), 8.16-8.21(m, 1H).

The product of Step 10 was converted to its methyl ester using CH₂N₂, and the ester was subjected to HPLC separation on chiral stationary phase (chiralcel OD column 2x25cm), eluting with 12% 2-propanol in hexane at a flow rate of 6 mL/min. Enantiomer A (less polar) has a retention time of 31.9 min and Enantiomer B (more polar) has a retention time of 35.5 min. Both A and B were hydrolyzed as in Ex. 17 Step 10 to give enantiomers A and B of the title compound.

### EXAMPLE 22

### ((1R)-6-Fluoro-8-(methylsulfonyl)-9-{(1S)-1-[4-(trifluoromethyl)phenyl]ethyl}-2,3,4,9-tetrahydro-1H-carbazol-1-yl)acetic acid (Compound AJ)

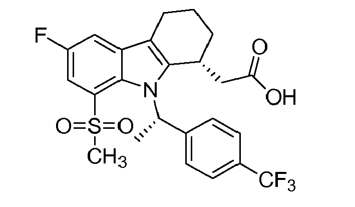

### Step 1: 2-(2-Bromo-4-fluorophenyl)hydrazinium chloride

To a suspension of 2-bromo-4-fluoroaniline in concentrated HCl (1.5M) at -10 °C was slowly added a 10.0M aqueous solution of NaNO₂ (1.1 eq). The mixture was stirred at 0 °C for 2.5 hrs. A cold (-30 °C) solution of SnCl₂ (3.8M) in concentrated HCl was then slowly added while maintaining the internal temperature below 10 °C. The resulting mixture was stirred mechanically for 20 min at 10 °C, then at room temperature for 1 hr. The thick slurry was filtered and the solid was air dried overnight. The solid was resuspended in cold HCl and filtered again. The dried material was suspended in Et₂O, stirred for 10 min, filtered and air dried overnight to give the title compound as a beige solid.

### Step 2: (+/-)-Ethyl (8-bromo-6-fluoro-2,3,4,9-tetrahydro-1H-carbazol-1-yl)acetate

To a suspension of the compound of Step 1 (1 eq) in AcOH (0.5M) was added ethyl (2-oxocyclohexyl)acetate (1 eq). The mixture was stirred at reflux for 16 hrs, cooled and AcOH was removed by evaporation under reduced pressure. The residue was diluted with EtOAc and washed with water and saturated aqueous NaHCO₃. The organic layer was dried over Na₂SO₄ and concentrated. The residue was then purified on a pad of silica gel, eluting with toluene. The filtrate was concentrated and stirred in hexanes to give, after filtration, the title compound as a white solid. MS (+APCI) m/z 354.2 (M+H)⁺.

### Step 3: (+/-) -Ethyl [6-fluoro-8-(methylsulfonyl)-2,3,4,9-tetrahydro-1H-carbazol-1-yl]-acetate

To a solution of the compound of Step 2 (1 eq) in anhydrous DMSO (0.28M) were added sodium methanesulphinate (3 eq) and copper iodide (3 eq). N₂ was bubbled into the mixture for 5 min and the reaction was then stirred at 100 °C under N₂ atmosphere. After 12 hrs, more sodium methanesulphinate (2 eq) and copper iodide (2 eq) were added. The mixture was stirred for a further 12hrs at 100 °C, cooled, diluted with EtOAc and 1N HCl was added to acidify the mixture. The suspension was stirred for 30 min and filtered through celite. The filtrate was washed with water, dried over Na₂SO₄ and concentrated. The residue was filtered through a pad of silica gel, eluting first with toluene to remove the non-polar impurities and then with a 2:1 mixture of hexanes/EtOAc to elute the desired product. The filtrate from the elution with the mixture of hexanes/EtOAc was concentrated to give the title compound as a pale yellow solid. MS (-APCI) m/z 352.1 (M-H)⁻.

### Step 4: Ethyl [(1R)-6-fluoro-8-(methylsulfonyl)-2,3,4,9-tetrahydro-1H-carbazol-1-yl]acetate

The racemic mixture from step 3 was resolved by preparative HPLC on a chiralpak AD preparative column eluted with a mixture of 15% iPrOH in hexane. The more polar enantiomer (longer retention time) was identified as the title compound based on the activity of the final product.

### Step 5: Ethyl [(1R)-9-[(1S)-1-(4-chlorophenyl)ethyl]-6-fluoro-8-(methylsulfonyl)-2,3,4,9-tetrahydro-1H-carbazol-1-yl]acetate

To a solution of the compound of Step 4 (1 eq), triphenylphosphine (1.5 eq) and (1R)-1-(4-chlorophenyl)ethanol (1.5 eq, prepared following the general procedure described in Reference Example 1) in THF (0.175M) was added a solution of di-tert-butyl azodicarboxylate (2.1 M in THF, 1.5 eq) over a 10 min period. The mixture was stirred at room temperature for 2hr and concentrated. The residue was purified by silica gel flash chromatography, eluting with 7% EtOAc in toluene to give the desired product (∼90% pure) which was used as such for the next reaction.

### Step 6: [(1R)-9-[(1S)-1-(4-Chlorophenyl)ethyl]-6-fluoro-8-(methylsulfonyl)-2,3,4,9-tetrahydro-1H-carbazol-1-yl]acetic acid and [(1S)-9-[(1S)-1-(4-chlorophenyl)ethyl]-6-fluoro-8-(methylsulfonyl)-2,3,4,9-tetrahydro-1H-carbazol-1-yl]acetic acid

To a solution of the compound of Step 5 in a 2:1 mixture of THF and methanol (0.1M) was added 1N aqueous LiOH (3 eq). The mixture was stirred at room temperature for 2 hr, AcOH was added and the solvent was removed by evaporation. The residue was taken up in EtOAc/H₂O and the organic layer was washed with brine, dried over Na₂SO₄, filtered and concentrated. The residue was swished in 30% EtOAc in hexane, and the product was suspended in diethyl ether and sonicated for 45 min, filtered, and dried under high vacuum at 50°C for 24 hr to give the title compound as a white solid. MS (-APCI) m/z 462.1 (M-H)

Alternatively (+/-) ethyl [6-fluoro-8-(methylsulfonyl)-2,3,4,9-tetrahydro-1H-carbazol-1-yl] acetate was used for the alkylation reaction in step 5 to give a mixture of 2 diastereomers: ethyl [(1R)-9-[(1S)-1-(4-chlorophenyl)ethyl]-6-fluoro-8-(methylsulfonyl)-2,3,4,9-tetrahydro-1H-carbazol-1-yl] acetate and ethyl [(1S)-9-[(1S)-1-(4-chlorophenyl)ethyl]-6-fluoro-8-(methylsulfonyl)-2,3,4,9-tetrahydro-1H-carbazol-1-yl]acetate. The diastereomeric mixture was resolved by selective hydrolysis using the following procedure to give the desired [(1R)-9-[(1S)-1-(4-chlorophenyl)ethyl]-6-fluoro-8-(methylsulfonyl)-2,3,4,9-tetrahydro-1H-carbazol-1-yl]acetic acid.

### Resolution:

The diastereomeric mixture of ethyl [(1R)-9-[(1S)-1-(4-chlorophenyl)ethyl]-6-fluoro-8-(methylsulfonyl)-2,3,4,9-tetrahydro-1H-carbazol-1-yl]acetate and ethyl [(1S)-9-[(1S)-1-(4-chlorophenyl)ethyl]-6-fluoro-8-(methylsulfonyl)-2,3,4,9-tetrahydro-1H-carbazol-1-yl]acetate (1 eq) was dissolved in a 3.5/1 mixture of THF /MeOH (0.25M) and cooled at 0°C. Aqueous LiOH 1N (1 eq) was slowly added and the mixture was stirred at 0°C for 12h or until almost complete hydrolysis of ethyl [(1R)-9-[(1S)-1-(4-chlorophenyl)ethyl]-6-fluoro-8-(methylsulfonyl)-2,3,4,9-tetrahydro-1 H-carbazol-1-yl] acetate, the other diastereomer was only slightly hydrolyzed under these conditions. AcOH was added and the solvent was removed by evaporation. The residue was taken up in EtOAc/H₂O and the organic layer was washed with brine, dried over Na₂SO₄, filtered and concentrated. Ethyl [(1S)-9-[(1S)-1-(4-chlorophenyl)-ethyl]-6-fluoro-8-(methylsulfonyl)-2,3,4,9-tetrahydro-1H-carbazol-1-yl]acetate and [(1R)-9-[(1S)-1-(4-chlorophenyl)ethyl]-6-fluoro-8-(methylsulfonyl)-2,3,4,9-tetrahydro-1H-carbazol-1-yl]acetic acid were separated by flash chromatography eluting with 40% EtOAc in hexanes containing 1% AcOH to give the desired [(1R)-9-[(1S)-1-(4-chlorophenyl)ethyl]-6-fluoro-8-(methylsulfonyl)-2,3,4,9-tetrahydro-1H-carbazol-1-yl]acetic acid with de>90% which was swished in 30% EtOAc in hexane to give the desired compound as a white solid with de>95%.

### Step 7: Methyl [(1R)-6-fluoro-8-(methylsulfonyl)-2,3,4,9-tetrahydro-1H-carbazol-1-yl]acetate

To a solution of [(1R)-9-[(1S)-1-(4-chlorophenyl)ethyl]-6-fluoro-8-(methylsulfonyl)-2,3,4,9-tetrahydro-1H-carbazol-1-yl]acetic acid ([α]_{D}= -226° in MeOH) in MeOH (0.1M) was added 10% palladium on carbon (10% wt/wt). A stream of N₂ was bubbled through the mixture for 5 min. The reaction was stirred at rt under H₂ atmosphere(balloon) for 24 hrs and filtered through a celite pad eluted with CH₂Cl₂. The solvents were removed by evaporation under reduced pressure and the residue was swished in MeOH to give the compound methyl [(1R)-6-fluoro-8-(methylsulfonyl)-2,3,4,9-tetrahydro-1H-carbazol-1-yl] acetate.

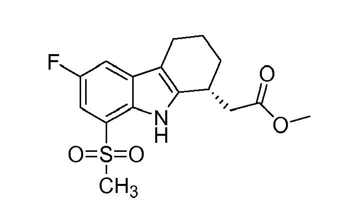

### Step 8: ((1R)-6-Fluoro-8-(methylsulfonyl)-9-{(1S)-1-[4-(trifluoromethyl)phenyl]ethyl}-2,3,4,9-tetrahydro-1H-carbazol-1-yl)acetic acid (Compound AJ)

To a solution of the compound of step 7 (1 eq), triphenylphosphine (1.5 eq) and (1R)-1-[4-(trifluoromethyl)phenyl]ethanol (1.5 eq) in THF (0.2M) was added a solution of di-tert-butyl azodicarboxylate (1M in THF, 1.5 eq) over a 20 min period. The mixture was stirred at room temperature for 2hr and concentrated. The residue was purified by silica gel flash chromatography eluted with 10% EtOAc in toluene to give methyl ((1R)-6-fluoro-8-(methylsulfonyl)-9-{(1S)-1-[4-(trifluoromethyl)phenyl]ethyl}-2,3,4,9-tetrahydro-1H-carbazol-1-yl)acetate (∼90% pure) which was used as such for the next reaction.

To a solution of the above ester (1 eq) in a 3.5/1 mixture of THF /MeOH (0.25M) at 0°C was slowly added aqueous LiOH 1N (1 eq) and the mixture was stirred at 0°C for 16h or until almost complete hydrolysis of the ester; under these conditions, the other minor diastereomer has a much slower rate of hydrolysis. AcOH was added and the solvent was removed in vacuo. The residue was taken up in EtOAc/H₂O and the organic layer was washed with brine, dried over Na_{z}SO₄, filtered and concentrated. To remove the unreacted methyl ester, the residue was filtered through a pad of silica gel eluting first with 10% EtOAc/toluene and then with 60% EtOAc/toluene containing 1% of AcOH. The residue was swished in 30% EtOAc/hexane and dried under high vacuum at 50°C for 16 hr to give the title compound as a white solid with de and ee >95% (checked by chiral HPLC). MS (-APCI) m/z 496.0 (M-H)⁻. [α]_{D}= -181° in MeOH

### Biology

The compounds used in the present invention that function as selective DP antagonists typically demonstrate an affinity (Kᵢ) for DP that is at least about 10 times higher (a numerically lower Kᵢ value) than the affinity (Kᵢ) for CRTH2 receptors. Typical DP antagonists used in the present invention are at least about 10-fold selective for the DP receptor over the CRTH2 receptor. More particularly, the selective DP receptor antagonist is at least about 100 fold selective for the DP receptor relative to the CRTH2 receptor. Even more particularly, the DP selective antagonist compound is at least about 800-1000 fold selective for the DP receptor over the CRTH2 receptor, i..e., the affinity (Kᵢ) for the DP receptor is 800-1000 times higher than the affinity (Kᵢ) for the CRTH2 receptor.

As used herein when a compound "selectively modulates the DP receptor", the compound binds to and antagonizes the DP receptor at a concentration that is achievable at therapeutic doses, while not substantially modulating the CRTH2 receptor at such therapeutically achievable concentrations.

Generally the DP antagonists used herein have an affinity (Kᵢ) for the CRTH2 receptor of about 0.5 micromolar or higher. Compounds having a binding affinity for CRTH2 of about 0.5 micromolar or higher, and a selectivity for the DP receptor over CRTH2 of at least about 10 fold, are useful to inhibit the flushing effect seen when nicotinic acid is administered without such selective DP antagonists.

### Determination of the Affinity and Selectivity of Compounds at Recombinant Human DP and CRTH2 Receptors

The receptor affinity and selectivity of compounds at DP and CRTH2 was determined using radioligand binding assays as described in Abramovitz M, et al. Biochem. Biophys. Acta (2000)1483: 285-293, and Sawyer N, et al. Br. J. Pharmacol. (2002); 137: 1163-1172. Briefly, stable cell lines that individually express human DP and CRTH2 receptors were established using human embryonic kidney (HEK) 293EBNA (Epstein Barr virus Nuclear Antigen) cells (designated HEK293E cell lines). Membrane fractions prepared from these recombinant cell lines were employed in equilibrium competition radioligand binding assays to determine the affinity and selectivity of compounds at the DP and CRTH2 receptors.

DP and CRTH2 cDNAs corresponding to full length coding sequences were subcloned into the appropriate sites of the mammalian expression vector pCEP4 (Invitrogen) and expressed in HEK293E cells. Membranes were prepared by differential centrifugation (1000 x g for 10 min, then 160,000 x g for 30 min, all at 4°C) following lysis of the cells by nitrogen cavitation at 800 psi for 30 min on ice in the presence of protease inhibitors (2 mM AEBSF, 10 µM E-64, 100 µM leupeptin and 0.05 mg/mL pepstatin). The 160,000 x g pellets were resuspended in 10 mM HEPES/KOH (pH 7.4) containing 1 mM EDTA at approximately 5 to 10 mg/mL protein by Dounce homogenisation (Dounce A; 10 strokes), frozen in liquid nitrogen and stored at -80°C. Receptor binding assays were performed in a final incubation volume of 0.2 mL in 10 mM HEPES/KOH (pH 7.4), containing 1 mM EDTA, 10 mM MnCl₂ and 0.7 nM [³H]PGD₂ (200 Ci/mmol). The reaction was initiated by addition of membrane protein (approximately 30 µg for DP and 10 µg for CRTH2) from the 160,000 x g fraction. Ligands were added in dimethylsulfoxide (DMSO) which was kept constant at 1 % (v/v) in all incubations. Non-specific binding was determined in the presence of 10 µM of non-radioactive PGD₂. Incubations were conducted on a mini-orbital shaker at room temperature for 60 min. The binding assay was terminated by rapid filtration through a 96-well Unifilter GF/C (Canberra Packard) prewetted in assay incubation buffer without EDTA (at 4°C) using a Tomtec Mach III 96-well semi-automated cell harvester. The filters were washed with 3 to 4 mL of the same buffer, dried for 90 min at 55°C and the residual radioactivity bound to the individual filters determined by scintillation counting with addition of 50 µL of Ultima Gold F (Canberra Packard) using a 1450 MicroBeta (Wallac) counter.

Maximum specific binding was defined as the total binding minus the non-specific binding in the absence of competitor. Specific binding was determined at each concentration of compound and was expressed as a percentage of the maximum specific binding. Sigmoidal equilibrium competition curves were constructed by expressing percentage maximum specific binding as a function of test compound concentration and analyzed by a custom designed software package employing a simplex driven non-linear least-squares curve fitting routine based on a four parameter equation to determine the inflection point (InPt). The binding affinity of the test compound was determined by calculating the equilibrium inhibition constant (Kᵢ) from the equation Kᵢ = InPt/1+([radioligand]/K_{d}), where K_{d} is the equilibrium dissociation constant for the radioligand-receptor interaction. When InPt could not be determined the IC₅₀ was used (i.e. the concentration of test compound required to inhibit 50 % of the maximum specific binding).

Generally the compounds used in the present invention demonstrate a Kᵢ for the DP receptor of from about as low as about 0.4 nM to as high as about 16.3 nM. Likewise, the compound used in the present invention generally demonstrate a Kᵢ for the CRTH2 receptor of as low as about 180 nM to as high as about 22,000 nM or even higher.

### Effect of Compounds on Nicotinic acid-Induced Vasodilation in Mice

The potency of the selective DP antagonists described herein can be demonstrated using a murine model of human nicotinic acid-induced flushing, measuring the flushing inhibitory effect. Blood flow in the mouse ear (a measure of vasodilation, a prominent component of flushing in humans) is measured after administration of nicotinic acid to mice that had been pretreated with vehicle (as a control) or a DP antagonist. Specifically, male C57BL/6 mice (∼25 g) were used in the study. Five mice were evaluated in each test group. Nembutal was diluted with water to a final concentration of 5 mg/ml and injected 0.3 ml/mouse intraperitoneally. DP antagonists were dissolved in 5% hydroxypropyl β-cyclodextrin at a final concentration of 5 mg/ml and the compounds were administered intraperitoneally at a volume of 0.2 ml/mouse (∼40 mpk). Nicotinic acid was dissolved in 5% hydroxypropyl β-cyclodextrin at a final concentration of 12.5 mg/ml. The nicotinic acid stock solution was adjusted to pH 7.4 with 2 N NaOH and injected 0.2 ml/mouse subcutaneously (∼100 mpk).

Perfusion of mouse ear skin was monitored with a laser Doppler perfusion imager (PeriScan PIM II, Perimed, Sweden) every 30 seconds for 15 minutes starting 5 minutes prior to nicotinic acid administration. Percent changes in mean perfusion over the 10 minute period after vehicle or nicotinic acid administration were calculated and a graph of percent change in mean perfusion vs. time was generated for each animal. The area under the curve (AUC) of mean perfusion (% Δ x min) was then calculated from each graph and the results are expressed in mean AUC ± SEM for each group.

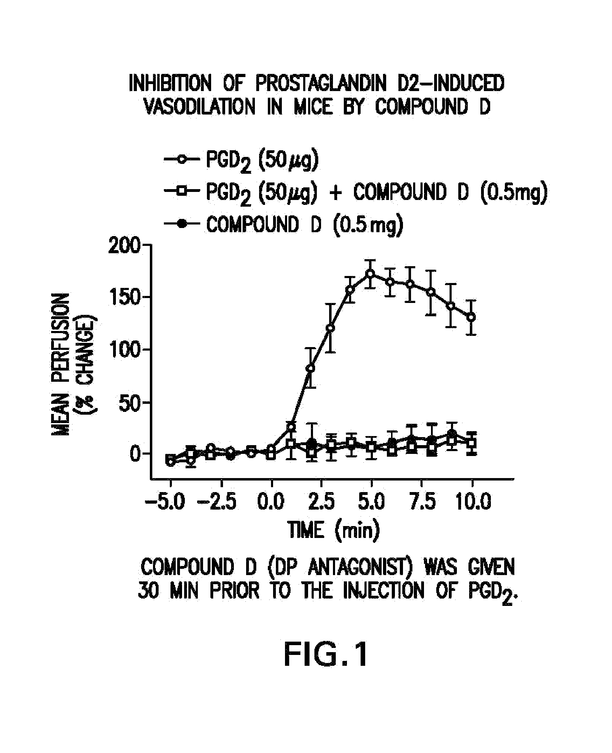

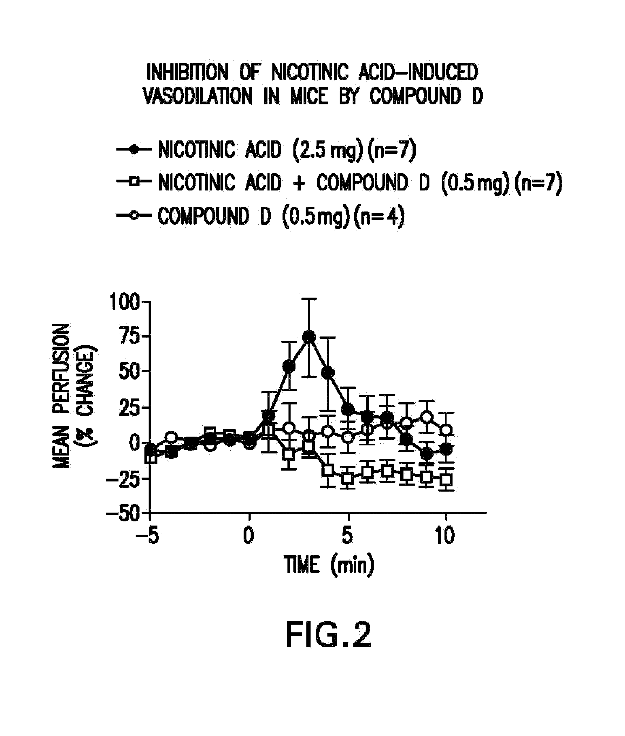

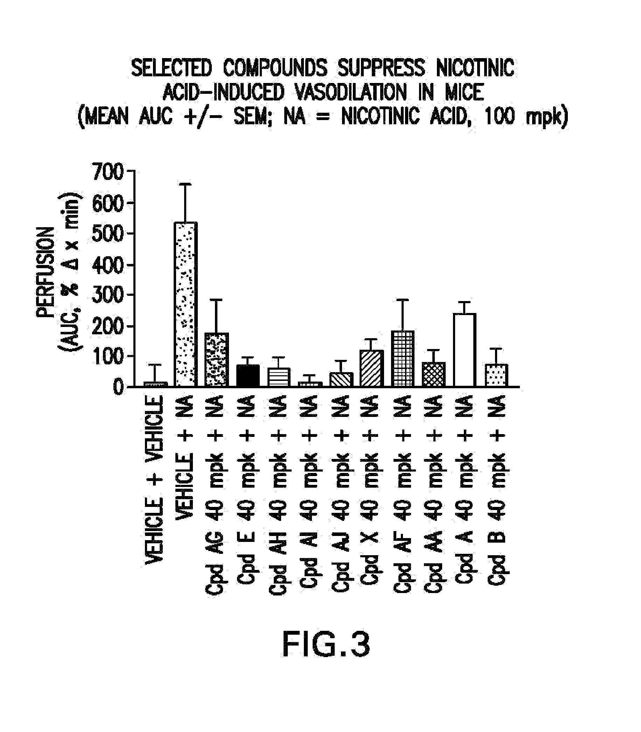

Compound D suppressed PGD-2 induced vasodilation in the mouse (Fig. 1). The DP antagonists tested suppressed nicotinic acid-induced vasodilation in the mouse; data for selected compounds is provided in Figures 2 and 3.

All patents, patent applications and publications that are cited herein are hereby incorporated by reference in their entirety. While certain preferred embodiments have been described herein in detail, numerous alternative embodiments are seen as falling within the scope of the invention.

## Claims

1. A combination of a DP antagonist and nicotinic acid for use in the treatment of atherosclerosis in the absence of substantial flushing; for use in the treatment of diabetes in the absence of substantial flushing; for use in raising serum HDL levels in the absence of substantial flushing; for use in treating dyslipidemia in the absence of substantial flushing; for use in reducing serum VLDL or LDL levels in the absence of substantial flushing; for use in reducing serum triglyceride levels in the absence of substantial flushing; or for use in reducing serum lipoprotein (a) levels in the absence of substantial flushing.

2. A combination of claim 1, further comprising one or more additional active ingredients.

3. A combination of claim 1, further comprising an HMG-CoA reductase inhibitor.

4. A combination of claim 3 wherein the HMG-CoA reductase inhibitor is selected from lovastatin, simvastatin, pravastatin, fluvastatin and atorvastatin.

5. A combination of any previous claim wherein the DP receptor antagonist is selected from: 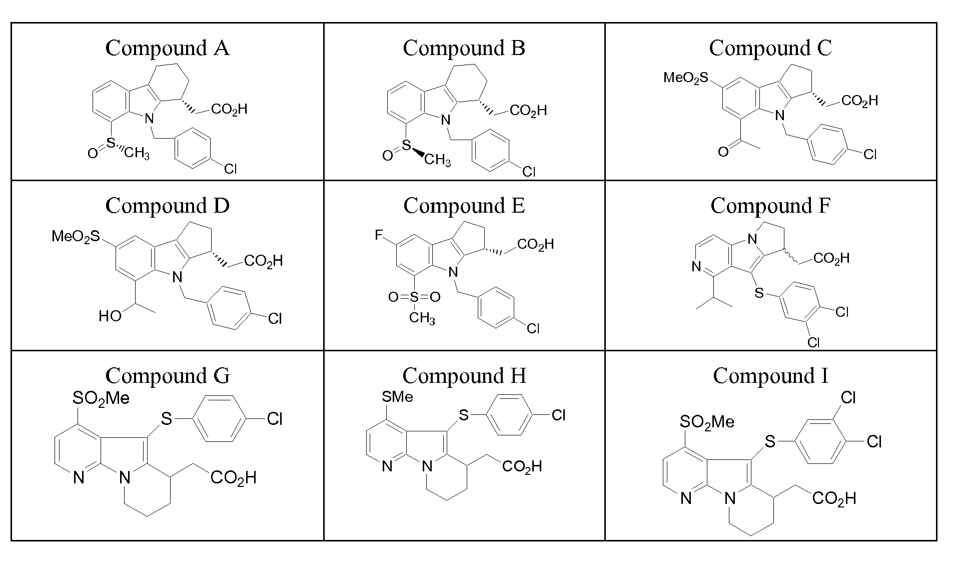 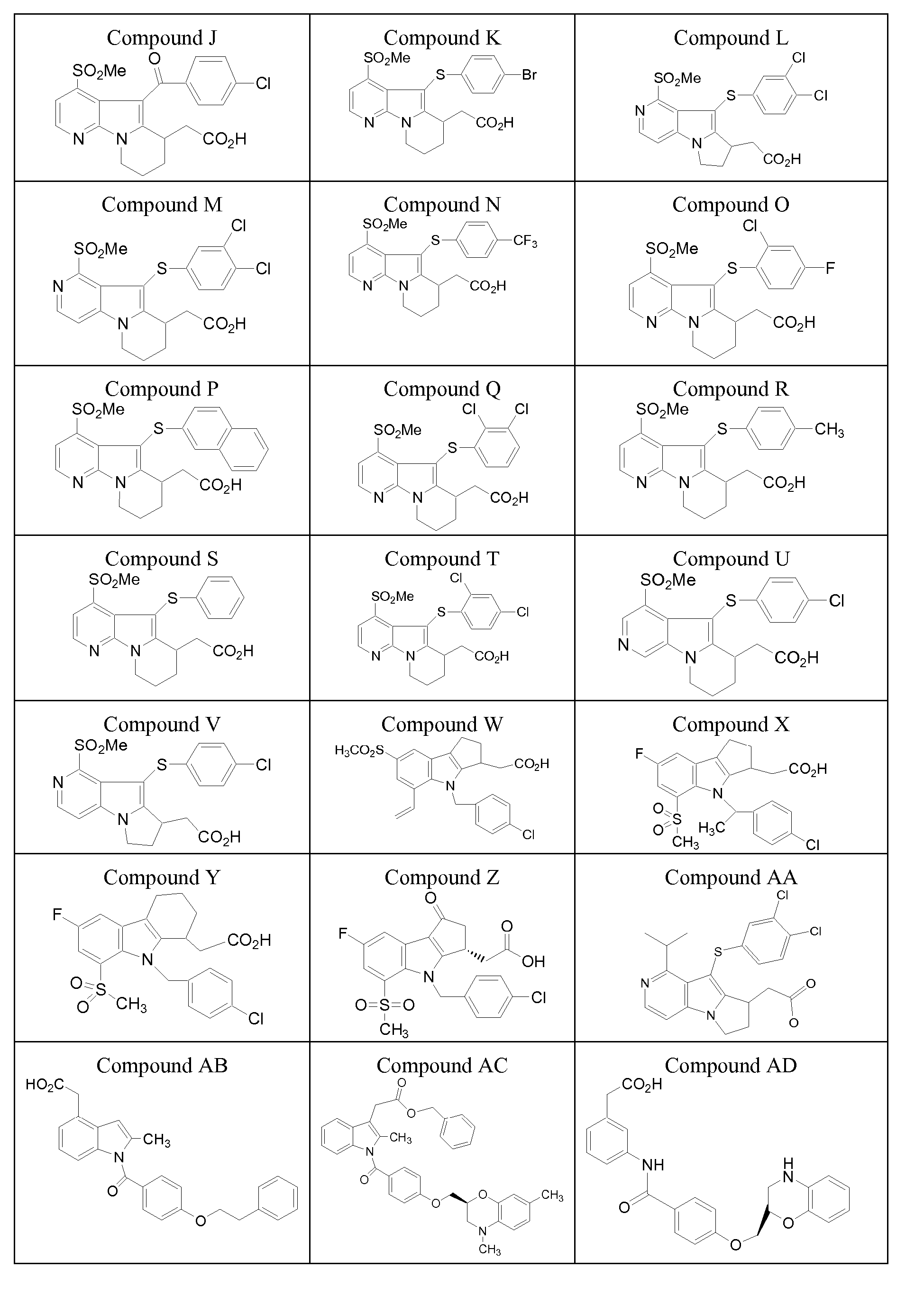 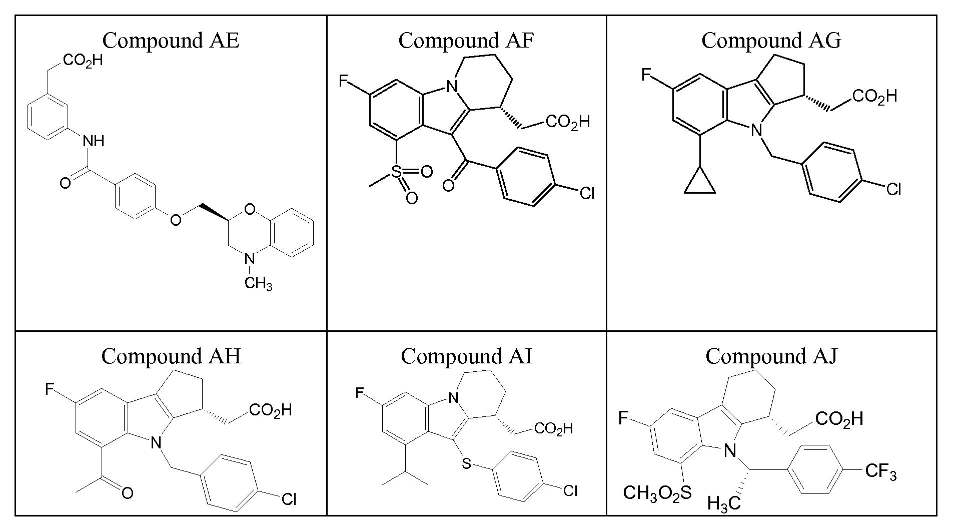 as well as the pharmaceutically acceptable salts and solvates thereof.

6. A compound of any previous claim wherein the DP antagonist is: 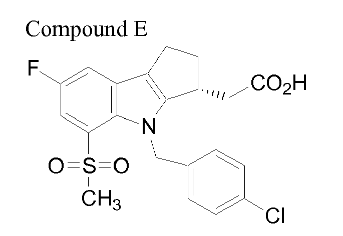 or a pharmaceutically acceptable salt or solvate thereof.

7. A combination of any previous claim wherein absence of substantial flushing is a reduced severity of flushing.

8. A combination of any previous claim wherein absence of substantial flushing is a reduction in the severity of flushing by at least a third.

9. A combination of any one of claims 1 to 6 wherein absence of substantial flushing is fewer flushing events.

10. A combination of any one of claims 1 to 6 and 9 wherein absence of substantial flushing is a reduction in the incidence of flushing by at least a third.
